# EUROPÄISCHE PATENTSCHRIFT

(11) **EP 2 728 355 B1**
(45) Veröffentlichungstag und Bekanntmachung des Hinweises auf die Patenterteilung: **22.02.2017**
(21) Anmeldenummer: 13191563.9
(22) Anmeldetag: 05.11.2013
(51) Int. Cl.: G01N 33/542, B01J 14/00, C07K 14/00

(54) **Verfahren und System zum direkten, strahlungslosen Energietransfer über flüssige Phasengrenzen**
Method and system for direct, radiation-free energy transfer across liquid phase boundaries
Procédé et système de transfert d'énergie direct sans rayonnement via des limites de phase liquides

(30) Priorität: 05.11.2012 DE 102012220083
(43) Veröffentlichungstag der Anmeldung: 07.05.2014
(73) Patentinhaber: Technische Universität Dresden, 01062 Dresden (DE)
(72) Erfinder: Gruner, Leopold, Dr., 01309 Dresden (DE); Rödel, Gerhard, Prof. Dr., 85757 Karlsfeld (DE); Ostermann, Kai, Dr., 01187 Dresden (DE); Eychmüller, Alexander, Prof. Dr., 01187 Dresden (DE); Bahrig, Lydia, 01129 Dresden (DE); Hickey, Stephen G., Dr., 01139 Dresden (DE)
(74) Vertreter: Kailuweit & Uhlemann Patentanwälte Partnerschaft mbB

(56) Entgegenhaltungen:
- EP-A2- 0 515 194
- KURZ ANKE ET AL: "Lipid-anchored oligonucleotides for stable double-helix formation in distinct membrane domains", ANGEWANDTE CHEMIE INTERNATIONAL EDITION, WILEY - V C H VERLAG GMBH & CO. KGAA, DE, Bd. 45, Nr. 27, 3. Juli 2006 (2006-07-03), Seiten 4440-4444, XP002459375, ISSN: 1433-7851, DOI: 10.1002/ANIE.200600822
- CHIARA NICOLINI ET AL: "Visualizing Association of N-Ras in Lipid Microdomains: Influence of Domain Structure and Interfacial Adsorption", JOURNAL OF THE AMERICAN CHEMICAL SOCIETY, Bd. 128, Nr. 1, 12. September 2005 (2005-09-12), Seiten 192-201, XP055098689, ISSN: 0002-7863, DOI: 10.1021/ja055779x
- J. J. JOENSUU ET AL: "Hydrophobin Fusions for High-Level Transient Protein Expression and Purification in Nicotiana benthamiana", PLANT PHYSIOLOGY, Bd. 152, Nr. 2, 1. Februar 2010 (2010-02-01), Seiten 622-633, XP055000691, ISSN: 0032-0889, DOI: 10.1104/pp.109.149021
- A. ORTIZ ET AL: "Fluorescence study on the interaction of a multiple antigenic peptide from hepatitis A virus with lipid vesicles", BIOPOLYMERS, Bd. 53, Nr. 6, 1. Mai 2000 (2000-05-01), Seiten 455-466, XP055098693, ISSN: 0006-3525, DOI: 10.1002/(SICI)1097-0282(200005)53:6<455::A ID-BIP2>3.3.CO;2-A
- SOEKARJO M ET AL: "THERMODYNAMICS OF THE MEMBRANE INSERTION PROCESS OF THE M13 PROCOAT PROTEIN, A LIPID BILAYER TRAVERSING PROTEIN CONTAINING A LEADER SEQUENCE", BIOCHEMISTRY, AMERICAN CHEMICAL SOCIETY, US, Bd. 35, Nr. 4, 1. Januar 1996 (1996-01-01) , Seiten 1232-1241, XP001083967, ISSN: 0006-2960, DOI: 10.1021/BI951087H
- Hedi Mattoussi ET AL: "Luminescent Quantum Dot-Bioconjugates in Immunoassays, FRET, Biosensing, and Imaging Applications", Journal of the Association for Laboratory Automation, Bd. 9 1. Februar 2004 (2004-02-01), Seiten 28-32, XP055098697, DOI: 10.1016/S1535-5535-03-00083-2 Gefunden im Internet: URL:http://jla.sagepub.com/content/9/1/28. full [gefunden am 2014-01-27]
- MCLEAN L R ET AL: "Kinetics of the interaction of amphipathic alpha-helical peptides with phosphatidylcholines", BIOCHIMICA ET BIOPHYSICA ACTA - LIPIDS AND LIPID METABOLISM, ELSEVIER SCIENCE BV. AMSTERDAM, NL, Bd. 1167, Nr. 3, 23. April 1993 (1993-04-23), Seiten 289-295, XP023365897, ISSN: 0005-2760, DOI: 10.1016/0005-2760(93)90231-W [gefunden am 1993-04-23]
- MANCHENO J M ET AL: "A PEPTIDE OF NINE AMINO ACID RESIDUES FROM ALPHA-SARCIN CYTOTOXIN IS A MEMBRANE-PERTURBING STRUCTURE", JOURNAL OF PEPTIDE RESEARCH, BLACKWELL PUBLISHING LTD, OXFORD; GB, Bd. 51, Nr. 2, 1. Februar 1998 (1998-02-01), Seiten 142-148, XP000730042, ISSN: 1397-002X
- WIMLEY W C ET AL: "Determining the membrane topology of peptides by fluorescence quenching", BIOCHEMISTRY, AMERICAN CHEMICAL SOCIETY, US, Bd. 39, Nr. 1, 11. Januar 2000 (2000-01-11), Seiten 161-170, XP002581605, ISSN: 0006-2960, DOI: 10.1021/BI9918361 [gefunden am 1999-12-09]
- GAE BAIK KIM: "Analysis of Protease Activity Using Quantum Dots and Resonance Energy Transfer", THERANOSTICS, Bd. 2, Nr. 2, 1. Februar 2012 (2012-02-01) , Seiten 127-138, XP055098701, ISSN: 1838-7640, DOI: 10.7150/thno.3476
- ISABEL COSTAS-MORA ET AL: "Quantum Dots Confined in an Organic Drop as Luminescent Probes for Detection of Selenium by Microfluorospectrometry after Hydridation: Study of the Quenching Mechanism and Analytical Performance", ANALYTICAL CHEMISTRY, Bd. 84, Nr. 10, 16. April 2012 (2012-04-16), Seiten 4452-4459, XP055098703, ISSN: 0003-2700, DOI: 10.1021/ac300221s
- C. Sato ET AL: "A Chemometric Approach to the Estimation of the Absorption Spectra of Dye Probe Merocyanine 540 in Aqueous and Phospholipid Environments", Journal of Biochemistry, vol. 127, no. 4, 1 April 2000 (2000-04-01) , pages 603-610, XP055308241, GB ISSN: 0021-924X, DOI: 10.1093/oxfordjournals.jbchem.a022647
- A. C. Cutró ET AL: "Phenylalanine Blocks Defects Induced in Gel Lipid Membranes by Osmotic Stress", Journal of Physical Chemistry Part B: Condensed Matter, Materials, Surfaces, Interfaces & Biophysical, vol. 119, no. 31, 6 August 2015 (2015-08-06), pages 10060-10065, XP055308242, US ISSN: 1520-6106, DOI: 10.1021/acs.jpcb.5b05590

### SEQUENCE LISTING

<110> Technische Universität Dresden
<120> Verfahren und System zum direkten, strahlungslosen Energietransfer über flüssige Phasengrenzen
<130> 00017P0211DEEP
<150> DE 10 2012 220 083.5
   <151> 2012-11-05
<160> 15
<170> PatentIn version 3.5
<210> 1
   <211> 41
   <212> PRT
   <213> Artificial Sequence
<220>
   <223> Chaplin Consensus Sequence
<220>
   <221> misc_feature
   <222> (4)..(5)
   <223> Xaa is selected from Val, Leu, Ile and Ala
<220>
   <221> misc_feature
   <222> (9)..(10)
   <223> Xaa is selected from Val, Leu, Ile, Thr, Ser and Ala
<220>
   <221> misc_feature
   <222> (12)..(12)
   <223> Xaa is selected from Val, Leu, Ile and Ala
<220>
   <221> misc_feature
   <222> (14)..(14)
   <223> XXaa is selected from Val, Leu, Ile and Ala
<220>
   <221> misc_feature
   <222> (15)..(15)
   <223> Xaa is selected from His, ASn and Asp
<220>
   <221> misc_feature
   <222> (16)..(16)
   <223> Xaa is selected from Val, Leu, Ile and Ala
<220>
   <221> misc_feature
   <222> (19) .. (19)
   <223> Xaa is selected from His, ASn and Asp
<220>
   <221> misc_feature
   <222> (20)..(20)
   <223> Xaa is selected from Val, Leu, Ile and Ala
<220>
   <221> misc_feature
   <222> (21) .. (21)
   <223> Xaa is selected from Cys, Val, Leu, Ile and Ala
<220>
   <221> misc_feature
   <222> (24) .. (24)
   <223> Xaa is selected from Ser und Thr
<220>
   <221> misc_feature
   <222> (25) .. (25)
   <223> Xaa is selected from Val, Leu, Ile and Ala
<220>
   <221> misc_feature
   <222> (26)..(26)
   <223> Xaa is selceted from Asn, Asp and Ser
<220>
   <221> misc_feature
   <222> (28)..(30)
   <223> Xaa are independently selected from Val, Leu, Ile and Ala
<220>
   <221> misc_feature
   <222> (35)..(35)
   <223> Xaa is selected from Ala, Phe and Met
<220>
   <221> misc_feature
   <222> (38)..(38)
   <223> Xaa can be any naturally occurring amino acid
<220>
   <221> misc_feature
   <222> (39) .. (39)
   <223> Xaa is selected from Gly and Cys
<220>
   <221> misc_feature
   <222> (40)..(40)
   <223> Xaa can be any naturally occurring amino acid
<220>
   <221> misc_feature
   <222> (41)..(41)
   <223> Xaa is selected from His, ASn and Asp
<400> 1
   Ser Pro Gly Xaa Xaa Ser Gly Asn Xaa Xaa Gln Xaa Pro Xaa Xaa Xaa
   1               5                   10                  15
   Pro Val Xaa Xaa Xaa Gly Asn Xaa Xaa Xaa Val Xaa Xaa Xaa Leu Asn
               20                  25                  30
   Pro Ala Xaa Gly Asn Xaa Xaa Xaa Xaa
                       35                  40
<210> 2
   <211> 1020
   <212> DNA
   <213> Artificial Sequence
<220>
   <223> Gene encoding fusion protein of class I hydrophobin CCg2 from
   Neurosporacrassa and turbo red fluorescent protein (tRFP)
<220>
   <221> CDS
   <222> (1)..(1020)
<400> 2
   atg ggc agc agc cat cat cat cat cat cac agc agc ggc ctg gtg ccg        48
   Met Gly Ser Ser His His His His His His Ser Ser Gly Leu Val Pro
   1               5                   10                  15
   cgc ggc agc cat atg atc ggc ccc aac acc tgc tcc atc gac gac tac        96
   Arg Gly Ser His Met Ile Gly Pro Asn Thr Cys Ser Ile Asp Asp Tyr
               20                  25                  30
   aag cct tac tgc tgc cag tct atg tcc ggc ccc gcc ggc tcc cct ggt       144
   Lys Pro Tyr Cys Cys Gln Ser Met Ser Gly Pro Ala Gly Ser Pro Gly
           35                  40                  45
   ctc ctc aac ctc atc ccc gtc gac ctc agc gcc tcg ctc ggc tgc gtt       192
   Leu Leu Asn Leu Ile Pro Val Asp Leu Ser Ala Ser Leu Gly Cys Val
       50                  55                  60
   gtc ggt gtc atc ggc tcc caa tgt ggt gcc agc gtc aag tgc tgc aag       240
   Val Gly Val Ile Gly Ser Gln Cys Gly Ala Ser Val Lys Cys Cys Lys
   65                  70                  75                  80
   gac gat gtt acc aac acc ggc aac tcc ttc ctc atc atc aac gct gcc       288
   Asp Asp Val Thr Asn Thr Gly Asn Ser Phe Leu Ile Ile Asn Ala Ala
                   85                  90                  95
   aac tgc gtt gcc gga ggc ggt ggg tct gga ggc ggc gga tca agc gag       336
   Asn Cys Val Ala Gly Gly Gly Gly Ser Gly Gly Gly Gly Ser Ser Glu
               100                 105                 110
   ctg atc aag gag aac atg cac atg aag ctg tac atg gag ggc acc gtg       384
   Leu Ile Lys Glu Asn Met His Met Lys Leu Tyr Met Glu Gly Thr Val
           115                 120                 125
   aac aac cac cac ttc aag tgc aca tcc gag ggc gaa ggc aag ccc tac       432
   Asn Asn His His Phe Lys Cys Thr Ser Glu Gly Glu Gly Lys Pro Tyr
       130                 135                 140
   gag ggc acc cag acc atg aag atc aag gtg gtc gag ggc ggc cct ctc       480
   Glu Gly Thr Gln Thr Met Lys Ile Lys Val Val Glu Gly Gly Pro Leu
   145                 150                 155                 160
   ccc ttc gcc ttc gac atc ctg gct acc agc ttc atg tac ggc agc aaa       528
   Pro Phe Ala Phe Asp Ile Leu Ala Thr Ser Phe Met Tyr Gly Ser Lys
                   165                 170                 175
   gcc ttc atc aac cac acc cag ggc atc ccc gac ttc ttt aag cag tcc        576
   Ala Phe Ile Asn His Thr Gln Gly Ile Pro Asp Phe Phe Lys Gln Ser
               180                 185                 190
   ttc cct gag ggc ttc aca tgg gag aga atc acc aca tac gaa gac ggg        624
   Phe Pro Glu Gly Phe Thr Trp Glu Arg Ile Thr Thr Tyr Glu Asp Gly
           195                 200                 205
   ggc gtg ctg acc gct acc cag gac acc agc ttc cag aac ggc tgc atc        672
   Gly Val Leu Thr Ala Thr Gln Asp Thr Ser Phe Gln Asn Gly Cys Ile
       210                 215                 220
   atc tac aac gtc aag atc aac ggg gtg aac ttc cca tcc aac ggc cct        720
   Ile Tyr Asn Val Lys Ile Asn Gly Val Asn Phe Pro Ser Asn Gly Pro
   225                 230                 235                 240
   gtg atg cag aag aaa aca cgc ggc tgg gag gcc aac acc gag atg ctg        768
   Val Met Gln Lys Lys Thr Arg Gly Trp Glu Ala Asn Thr Glu Met Leu
                   245                 250                 255
   tac ccc gct gac ggc ggc ctg aga ggc cac agc cag atg gcc ctg aag        816
   Tyr Pro Ala Asp Gly Gly Leu Arg Gly His Ser Gln Met Ala Leu Lys
               260                 265                 270
   ctc gtg ggc ggg ggc tac ctg cac tgc tcc ttc aag acc aca tac aga        864
   Leu Val Gly Gly Gly Tyr Leu His Cys Ser Phe Lys Thr Thr Tyr Arg
           275                 280                 285
   tcc aag aaa ccc gct aag aac ctc aag atg ccc ggc ttc cac ttc gtg        912
   Ser Lys Lys Pro Ala Lys Asn Leu Lys Met Pro Gly Phe His Phe Val
       290                 295                 300
   gac cac aga ctg gaa aga atc aag gag gcc gac aaa gag acc tac gtc        960
   Asp His Arg Leu Glu Arg Ile Lys Glu Ala Asp Lys Glu Thr Tyr Val
   305                 310                 315                 320
   gag cag cac gag atg gct gtg gcc aag tac tgc gac ctc cct agc aaa       1008
   Glu Gln His Glu Met Ala Val Ala Lys Tyr Cys Asp Leu Pro Ser Lys
                   325                 330                 335
   ctg ggg cac aga                                                       1020
   Leu Gly His Arg
               340
<210> 3
   <211> 340
   <212> PRT
   <213> Artificial Sequence
<220>
   <223> Fusion protein of class I hydrophobin CCg2 from Neurosporacrassa and turbo red fluorescent protein (tRFP)
<400> 3
   Met Gly Ser Ser His His His His His His Ser Ser Gly Leu Val Pro
   1               5                   10                  15
   Arg Gly Ser His Met Ile Gly Pro Asn Thr Cys Ser Ile Asp Asp Tyr
               20                  25                  30
   Lys Pro Tyr Cys Cys Gln Ser Met Ser Gly Pro Ala Gly Ser Pro Gly
           35                  40                  45
   Leu Leu Asn Leu Ile Pro Val Asp Leu Ser Ala Ser Leu Gly Cys Val
       50                  55                  60
   Val Gly Val Ile Gly Ser Gln Cys Gly Ala Ser Val Lys Cys Cys Lys
   65                  70                  75                  80
   Asp Asp Val Thr Asn Thr Gly Asn Ser Phe Leu Ile Ile Asn Ala Ala
                   85                  90                  95
   Asn Cys Val Ala Gly Gly Gly Gly Ser Gly Gly Gly Gly Ser Ser Glu
               100                 105                 110
   Leu Ile Lys Glu Asn Met His Met Lys Leu Tyr Met Glu Gly Thr Val
           115                 120                 125
   Asn Asn His His Phe Lys Cys Thr Ser Glu Gly Glu Gly Lys Pro Tyr
       130                 135                 140
   Glu Gly Thr Gln Thr Met Lys Ile Lys Val Val Glu Gly Gly Pro Leu
   145                 150                 155                 160
   Pro Phe Ala Phe Asp Ile Leu Ala Thr Ser Phe Met Tyr Gly Ser Lys
                   165                 170                 175
   Ala Phe Ile Asn His Thr Gln Gly Ile Pro Asp Phe Phe Lys Gln Ser
               180                 185                 190
   Phe Pro Glu Gly Phe Thr Trp Glu Arg Ile Thr Thr Tyr Glu Asp Gly
           195                 200                 205
   Gly Val Leu Thr Ala Thr Gln Asp Thr Ser Phe Gln Asn Gly Cys Ile
       210                 215                 220
   Ile Tyr Asn Val Lys Ile Asn Gly Val Asn Phe Pro Ser Asn Gly Pro
   225                 230                 235                 240
   Val Met Gln Lys Lys Thr Arg Gly Trp Glu Ala Asn Thr Glu Met Leu
                   245                 250                 255
   Tyr Pro Ala Asp Gly Gly Leu Arg Gly His Ser Gln Met Ala Leu Lys
               260                 265                 270
   Leu Val Gly Gly Gly Tyr Leu His Cys Ser Phe Lys Thr Thr Tyr Arg
           275                 280                 285
   Ser Lys Lys Pro Ala Lys Asn Leu Lys Met Pro Gly Phe His Phe Val
       290                 295                 300
   Asp His Arg Leu Glu Arg Ile Lys Glu Ala Asp Lys Glu Thr Tyr Val
   305                 310                 315                 320
   Glu Gln His Glu Met Ala Val Ala Lys Tyr Cys Asp Leu Pro Ser Lys
                   325                 330                 335
   Leu Gly His Arg
               340
<210> 4
   <211> 1005
   <212> DNA
   <213> Artificial Sequence
<220>
   <223> Gene encoding fusion protein of class II hydrophobin HFBI from
   Trichoderma reesei and enhanced green fluorescent protein (eGFP)
<220>
   <221> CDS
   <222> (1)..(1005)
<400> 4
   atg ggc agc agc cat cat cat cat cat cac agc agc ggc ctg gtg ccg      48
   Met Gly Ser Ser His His His His His His Ser Ser Gly Leu Val Pro
   1               5                   10                  15
   cgc ggc agc cat atg agc aac ggc aac ggc aat gtt tgc cct ccc ggc      96
   Arg Gly Ser His Met Ser Asn Gly Asn Gly Asn Val Cys Pro Pro Gly
               20                  25                  30
   ctc ttc agc aac ccc cag tgc tgt gcc acc caa gtc ctt ggc ctc atc     144
   Leu Phe Ser Asn Pro Gln Cys Cys Ala Thr Gln Val Leu Gly Leu Ile
           35                  40                  45
   ggc ctt gac tgc aaa gtc ccc tcc cag aac gtt tac gac ggc acc gac     192
   Gly Leu Asp Cys Lys Val Pro Ser Gln Asn Val Tyr Asp Gly Thr Asp
       50                  55                  60
   ttc cgc aac gtc tgc gcc aaa acc ggc gcc cag cct ctc tgc tgc gtg     240
   Phe Arg Asn Val Cys Ala Lys Thr Gly Ala Gln Pro Leu Cys Cys Val
   65                  70                  75                  80
   gcc ccc gtt gcc ggc cag gct ctt ctg tgc cag acc gcc gtc ggt gct     288
   Ala Pro Val Ala Gly Gln Ala Leu Leu Cys Gln Thr Ala Val Gly Ala
                   85                  90                  95
   atg gtg agc aag ggc gag gag ctg ttc acc ggg gtg gtg ccc atc ctg     336
   Met Val Ser Lys Gly Glu Glu Leu Phe Thr Gly Val Val Pro Ile Leu
               100                 105                 110
   gtc gag ctg gac ggc gac gta aac ggc cac aag ttc agc gtg tcc ggc     384
   Val Glu Leu Asp Gly Asp Val Asn Gly His Lys Phe Ser Val Ser Gly
           115                 120                 125
   gag ggc gag ggc gat gcc acc tac ggc aag ctg acc ctg aag ttc atc        432
   Glu Gly Glu Gly Asp Ala Thr Tyr Gly Lys Leu Thr Leu Lys Phe Ile
       130                 135                 140
   tgc acc acc ggc aag ctg ccc gtg ccc tgg ccc acc ctc gtg acc acc        480
   Cys Thr Thr Gly Lys Leu Pro Val Pro Trp Pro Thr Leu Val Thr Thr
   145                 150                 155                 160
   ctg acc tac ggc gtg cag tgc ttc agc cgc tac ccc gac cac atg aag        528
   Leu Thr Tyr Gly Val Gln Cys Phe Ser Arg Tyr Pro Asp His Met Lys
                   165                 170                 175
   cag cac gac ttc ttc aag tcc gcc atg ccc gaa ggc tac gtc cag gag        576
   Gln His Asp Phe Phe Lys Ser Ala Met Pro Glu Gly Tyr Val Gln Glu
               180                 185                 190
   cgc acc atc ttc ttc aag gac gac ggc aac tac aag acc cgc gcc gag        624
   Arg Thr Ile Phe Phe Lys Asp Asp Gly Asn Tyr Lys Thr Arg Ala Glu
           195                 200                 205
   gtg aag ttc gag ggc gac acc ctg gtg aac cgc atc gag ctg aag ggc        672
   Val Lys Phe Glu Gly Asp Thr Leu Val Asn Arg Ile Glu Leu Lys Gly
       210                 215                 220
   atc gac ttc aag gag gac ggc aac atc ctg ggg cac aag ctg gag tac        720
   Ile Asp Phe Lys Glu Asp Gly Asn Ile Leu Gly His Lys Leu Glu Tyr
   225                 230                 235                 240
   aac tac aac agc cac aac gtc tat atc atg gcc gac aag cag aag aac        768
   Asn Tyr Asn Ser His Asn Val Tyr Ile Met Ala Asp Lys Gln Lys Asn
                   245                 250                 255
   ggc atc aag gtg aac ttc aag atc cgc cac aac atc gag gac ggc agc        816
   Gly Ile Lys Val Asn Phe Lys Ile Arg His Asn Ile Glu Asp Gly Ser
               260                 265                 270
   gtg cag ctc gcc gac cac tac cag cag aac acc ccc atc ggc gac ggc        864
   Val Gln Leu Ala Asp His Tyr Gln Gln Asn Thr Pro Ile Gly Asp Gly
           275                 280                 285
   ccc gtg ctg ctg ccc gac aac cac tac ctg agc acc cag tcc gcc ctg        912
   Pro Val Leu Leu Pro Asp Asn His Tyr Leu Ser Thr Gln Ser Ala Leu
       290                 295                 300
   agc aaa gac ccc aac gag aag cgc gat cac atg gtc ctg ctg gag ttc        960
   Ser Lys Asp Pro Asn Glu Lys Arg Asp His Met Val Leu Leu Glu Phe
   305                 310                 315                 320
   gtg acc gcc gcc ggg atc act ctc ggc atg gac gag ctg tac aag           1005
   Val Thr Ala Ala Gly Ile Thr Leu Gly Met Asp Glu Leu Tyr Lys
                   325                 330                 335
<210> 5
   <211> 335
   <212> PRT
   <213> Artificial Sequence
<220>
   <223> Fusion protein of class II hydrophobin HFBI from Trichoderma reesei and enhanced green fluorescent protein (eGFP)
<400> 5
   Met Gly Ser Ser His His His His His His Ser Ser Gly Leu Val Pro
   1               5                   10                  15
   Arg Gly Ser His Met Ser Asn Gly Asn Gly Asn Val Cys Pro Pro Gly
               20                  25                  30
   Leu Phe Ser Asn Pro Gln Cys Cys Ala Thr Gln Val Leu Gly Leu Ile
           35                  40                  45
   Gly Leu Asp Cys Lys Val Pro Ser Gln Asn Val Tyr Asp Gly Thr Asp
       50                  55                  60
   Phe Arg Asn Val Cys Ala Lys Thr Gly Ala Gln Pro Leu Cys Cys Val
   65                  70                  75                  80
   Ala Pro Val Ala Gly Gln Ala Leu Leu Cys Gln Thr Ala Val Gly Ala
                   85                  90                  95
   Met Val Ser Lys Gly Glu Glu Leu Phe Thr Gly Val Val Pro Ile Leu
               100                 105                 110
   Val Glu Leu Asp Gly Asp Val Asn Gly His Lys Phe Ser Val Ser Gly
           115                 120                 125
   Glu Gly Glu Gly Asp Ala Thr Tyr Gly Lys Leu Thr Leu Lys Phe Ile
       130                 135                 140
   Cys Thr Thr Gly Lys Leu Pro Val Pro Trp Pro Thr Leu Val Thr Thr
   145                 150                 155                 160
   Leu Thr Tyr Gly Val Gln Cys Phe Ser Arg Tyr Pro Asp His Met Lys
                   165                 170                 175
   Gln His Asp Phe Phe Lys Ser Ala Met Pro Glu Gly Tyr Val Gln Glu
               180                 185                 190
   Arg Thr Ile Phe Phe Lys Asp Asp Gly Asn Tyr Lys Thr Arg Ala Glu
           195                 200                 205
   Val Lys Phe Glu Gly Asp Thr Leu Val Asn Arg Ile Glu Leu Lys Gly
       210                 215                 220
   Ile Asp Phe Lys Glu Asp Gly Asn Ile Leu Gly His Lys Leu Glu Tyr
   225                 230                 235                 240
   Asn Tyr Asn Ser His Asn Val Tyr Ile Met Ala Asp Lys Gln Lys Asn
                   245                 250                 255
   Gly Ile Lys Val Asn Phe Lys Ile Arg His Asn Ile Glu Asp Gly Ser
               260                 265                 270
   Val Gln Leu Ala Asp His Tyr Gln Gln Asn Thr Pro Ile Gly Asp Gly
           275                 280                 285
   Pro Val Leu Leu Pro Asp Asn His Tyr Leu Ser Thr Gln Ser Ala Leu
       290                 295                 300
   Ser Lys Asp Pro Asn Glu Lys Arg Asp His Met Val Leu Leu Glu Phe
   305                 310                 315                 320
   Val Thr Ala Ala Gly Ile Thr Leu Gly Met Asp Glu Leu Tyr Lys
                   325                 330                 335
<210> 6
   <211> 1008
   <212> DNA
   <213> Artificial Sequence
<220>
   <223> Gene encoding fusion protein of class II hydrophobin HFBI from Trichoderma reesei and turbo red fluorescent protein (tRFP)
<220>
   <221> CDS
   <222> (1)..(1008)
<400> 6
   atg ggc agc agc cat cat cat cat cat cac agc agc ggc ctg gtg ccg       48
   Met Gly Ser Ser His His His His His His Ser Ser Gly Leu Val Pro
   1               5                   10                  15
   cgc ggc agc cat atg agc aac ggc aac ggc aat gtt tgc cct ccc ggc       96
   Arg Gly Ser His Met Ser Asn Gly Asn Gly Asn Val Cys Pro Pro Gly
               20                  25                  30
   ctc ttc agc aac ccc cag tgc tgt gcc acc caa gtc ctt ggc ctc atc      144
   Leu Phe Ser Asn Pro Gln Cys Cys Ala Thr Gln Val Leu Gly Leu Ile
           35                  40                  45
   ggc ctt gac tgc aaa gtc ccc tcc cag aac gtt tac gac ggc acc gac      192
   Gly Leu Asp Cys Lys Val Pro Ser Gln Asn Val Tyr Asp Gly Thr Asp
       50                  55                  60
   ttc cgc aac gtc tgc gcc aaa acc ggc gcc cag cct ctc tgc tgc gtg      240
   Phe Arg Asn Val Cys Ala Lys Thr Gly Ala Gln Pro Leu Cys Cys Val
   65                  70                  75                  80
   gcc ccc gtt gcc ggc cag gct ctt ctg tgc cag acc gcc gtc ggt gct      288
   Ala Pro Val Ala Gly Gln Ala Leu Leu Cys Gln Thr Ala Val Gly Ala
                   85                  90                  95
   gga ggc ggt ggg tct gga ggc ggc gga tca agc gag ctg atc aag gag      336
   Gly Gly Gly Gly Ser Gly Gly Gly Gly Ser Ser Glu Leu Ile Lys Glu
               100                 105                 110
   aac atg cac atg aag ctg tac atg gag ggc acc gtg aac aac cac cac      384
   Asn Met His Met Lys Leu Tyr Met Glu Gly Thr Val Asn Asn His His
           115                 120                 125
   ttc aag tgc aca tcc gag ggc gaa ggc aag ccc tac gag ggc acc cag      432
   Phe Lys Cys Thr Ser Glu Gly Glu Gly Lys Pro Tyr Glu Gly Thr Gln
       130                 135                 140
   acc atg aag atc aag gtg gtc gag ggc ggc cct ctc ccc ttc gcc ttc      480
   Thr Met Lys Ile Lys Val Val Glu Gly Gly Pro Leu Pro Phe Ala Phe
   145                 150                 155                 160
   gac atc ctg gct acc agc ttc atg tac ggc agc aaa gcc ttc atc aac      528
   Asp Ile Leu Ala Thr Ser Phe Met Tyr Gly Ser Lys Ala Phe Ile Asn
                   165                 170                 175
   cac acc cag ggc atc ccc gac ttc ttt aag cag tcc ttc cct gag ggc      576
   His Thr Gln Gly Ile Pro Asp Phe Phe Lys Gln Ser Phe Pro Glu Gly
               180                 185                 190
   ttc aca tgg gag aga atc acc aca tac gaa gac ggg ggc gtg ctg acc      624
   Phe Thr Trp Glu Arg Ile Thr Thr Tyr Glu Asp Gly Gly Val Leu Thr
           195                 200                 205
   gct acc cag gac acc agc ttc cag aac ggc tgc atc atc tac aac gtc      672
   Ala Thr Gln Asp Thr Ser Phe Gln Asn Gly Cys Ile Ile Tyr Asn Val
       210                 215                 220
   aag atc aac ggg gtg aac ttc cca tcc aac ggc cct gtg atg cag aag      720
   Lys Ile Asn Gly Val Asn Phe Pro Ser Asn Gly Pro Val Met Gln Lys
   225                 230                 235                 240
   aaa aca cgc ggc tgg gag gcc aac acc gag atg ctg tac ccc gct gac      768
   Lys Thr Arg Gly Trp Glu Ala Asn Thr Glu Met Leu Tyr Pro Ala Asp
                   245                 250                 255
   ggc ggc ctg aga ggc cac agc cag atg gcc ctg aag ctc gtg ggc ggg      816
   Gly Gly Leu Arg Gly His Ser Gln Met Ala Leu Lys Leu Val Gly Gly
               260                 265                 270
   ggc tac ctg cac tgc tcc ttc aag acc aca tac aga tcc aag aaa ccc      864
   Gly Tyr Leu His Cys Ser Phe Lys Thr Thr Tyr Arg Ser Lys Lys Pro
           275                 280                 285
   gct aag aac ctc aag atg ccc ggc ttc cac ttc gtg gac cac aga ctg      912
   Ala Lys Asn Leu Lys Met Pro Gly Phe His Phe Val Asp His Arg Leu
       290                 295                 300
   gaa aga atc aag gag gcc gac aaa gag acc tac gtc gag cag cac gag      960
   Glu Arg Ile Lys Glu Ala Asp Lys Glu Thr Tyr Val Glu Gln His Glu
   305                 310                 315                 320
   atg gct gtg gcc aag tac tgc gac ctc cct agc aaa ctg ggg cac aga     1008
   Met Ala Val Ala Lys Tyr Cys Asp Leu Pro Ser Lys Leu Gly His Arg
                   325                 330                 335
<210> 7
   <211> 336
   <212> PRT
   <213> Artificial Sequence
<220>
   <223> Fusion protein of class II hydrophobin HFBI from Trichoderma reesei and turbo red fluorescent protein (tRFP)
<400> 7
   Met Gly Ser Ser His His His His His His Ser Ser Gly Leu Val Pro
   1               5                   10                  15
   Arg Gly Ser His Met Ser Asn Gly Asn Gly Asn Val Cys Pro Pro Gly
               20                  25                  30
   Leu Phe Ser Asn Pro Gln Cys Cys Ala Thr Gln Val Leu Gly Leu Ile
           35                  40                  45
   Gly Leu Asp Cys Lys Val Pro Ser Gln Asn Val Tyr Asp Gly Thr Asp
       50                  55                  60
   Phe Arg Asn Val Cys Ala Lys Thr Gly Ala Gln Pro Leu Cys Cys Val
   65                  70                  75                  80
   Ala Pro Val Ala Gly Gln Ala Leu Leu Cys Gln Thr Ala Val Gly Ala
                   85                  90                  95
   Gly Gly Gly Gly Ser Gly Gly Gly Gly Ser Ser Glu Leu Ile Lys Glu
               100                 105                 110
   Asn Met His Met Lys Leu Tyr Met Glu Gly Thr Val Asn Asn His His
           115                 120                 125
   Phe Lys Cys Thr Ser Glu Gly Glu Gly Lys Pro Tyr Glu Gly Thr Gln
       130                 135                 140
   Thr Met Lys Ile Lys Val Val Glu Gly Gly Pro Leu Pro Phe Ala Phe
   145                 150                 155                 160
   Asp Ile Leu Ala Thr Ser Phe Met Tyr Gly Ser Lys Ala Phe Ile Asn
                   165                 170                 175
   His Thr Gln Gly Ile Pro Asp Phe Phe Lys Gln Ser Phe Pro Glu Gly
               180                 185                 190
   Phe Thr Trp Glu Arg Ile Thr Thr Tyr Glu Asp Gly Gly Val Leu Thr
           195                 200                 205
   Ala Thr Gln Asp Thr Ser Phe Gln Asn Gly Cys Ile Ile Tyr Asn Val
               210                 215                 220
   Lys Ile Asn Gly Val Asn Phe Pro Ser Asn Gly Pro Val Met Gln Lys
   225                 230                 235                 240
   Lys Thr Arg Gly Trp Glu Ala Asn Thr Glu Met Leu Tyr Pro Ala Asp
                   245                 250                 255
   Gly Gly Leu Arg Gly His Ser Gln Met Ala Leu Lys Leu Val Gly Gly
               260                 265                 270
   Gly Tyr Leu His Cys Ser Phe Lys Thr Thr Tyr Arg Ser Lys Lys Pro
           275                 280                 285
   Ala Lys Asn Leu Lys Met Pro Gly Phe His Phe Val Asp His Arg Leu
       290                 295                 300
   Glu Arg Ile Lys Glu Ala Asp Lys Glu Thr Tyr Val Glu Gln His Glu
   305                 310                 315                 320
   Met Ala Val Ala Lys Tyr Cys Asp Leu Pro Ser Lys Leu Gly His Arg
                   325                 330                 335
<210> 8
   <211> 1152
   <212> DNA
   <213> Artificial Sequence
<220>
   <223> Gene encoding fusion protein of class II hydrophobin, artifical polypeptide ArtOLEO and turbo red fluorescent protein (tRFP)
<220>
   <221> CDS
   <222> (1)..(1152)
<400> 8
   atg gct agc agc gag ctg atc aag gag aac atg cac atg aag ctg tac       48
   Met Ala Ser Ser Glu Leu Ile Lys Glu Asn Met His Met Lys Leu Tyr
   1               5                   10                  15
   atg gag ggc acc gtg aac aac cac cac ttc aag tgc aca tcc gag ggc       96
   Met Glu Gly Thr Val Asn Asn His His Phe Lys Cys Thr Ser Glu Gly
               20                  25                  30
   gaa ggc aag ccc tac gag ggc acc cag acc atg aag atc aag gtg gtc      144
   Glu Gly Lys Pro Tyr Glu Gly Thr Gln Thr Met Lys Ile Lys Val Val
           35                  40                  45
   gag ggc ggc cct ctc ccc ttc gcc ttc gac atc ctg gct acc agc ttc      192
   Glu Gly Gly Pro Leu Pro Phe Ala Phe Asp Ile Leu Ala Thr Ser Phe
       50                  55                  60
   atg tac ggc agc aaa gcc ttc atc aac cac acc cag ggc atc ccc gac         240
   Met Tyr Gly Ser Lys Ala Phe Ile Asn His Thr Gln Gly Ile Pro Asp
   65                  70                  75                  80
   ttc ttt aag cag tcc ttc cct gag ggc ttc aca tgg gag aga atc acc         288
   Phe Phe Lys Gln Ser Phe Pro Glu Gly Phe Thr Trp Glu Arg Ile Thr
                   85                  90                  95
   aca tac gaa gac ggg ggc gtg ctg acc gct acc cag gac acc agc ttc         336
   Thr Tyr Glu Asp Gly Gly Val Leu Thr Ala Thr Gln Asp Thr Ser Phe
               100                 105                 110
   cag aac ggc tgc atc atc tac aac gtc aag atc aac ggg gtg aac ttc         384
   Gln Asn Gly Cys Ile Ile Tyr Asn Val Lys Ile Asn Gly Val Asn Phe
           115                 120                 125
   cca tcc aac ggc cct gtg atg cag aag aaa aca cgc ggc tgg gag gcc         432
   Pro Ser Asn Gly Pro Val Met Gln Lys Lys Thr Arg Gly Trp Glu Ala
       130                 135                 140
   aac acc gag atg ctg tac ccc gct gac ggc ggc ctg aga ggc cac agc         480
   Asn Thr Glu Met Leu Tyr Pro Ala Asp Gly Gly Leu Arg Gly His Ser
   145                 150                 155                 160
   cag atg gcc ctg aag ctc gtg ggc ggg ggc tac ctg cac tgc tcc ttc         528
   Gln Met Ala Leu Lys Leu Val Gly Gly Gly Tyr Leu His Cys Ser Phe
                   165                 170                 175
   aag acc aca tac aga tcc aag aaa ccc gct aag aac ctc aag atg ccc         576
   Lys Thr Thr Tyr Arg Ser Lys Lys Pro Ala Lys Asn Leu Lys Met Pro
               180                 185                 190
   ggc ttc cac ttc gtg gac cac aga ctg gaa aga atc aag gag gcc gac         624
   Gly Phe His Phe Val Asp His Arg Leu Glu Arg Ile Lys Glu Ala Asp
           195                 200                 205
   aaa gag acc tac gtc gag cag cac gag atg gct gtg gcc aag tac tgc         672
   Lys Glu Thr Tyr Val Glu Gln His Glu Met Ala Val Ala Lys Tyr Cys
       210                 215                 220
   gac ctc cct agc aaa ctg ggg cac aga gga ggc ggt ggg tct gga ggc         720
   Asp Leu Pro Ser Lys Leu Gly His Arg Gly Gly Gly Gly Ser Gly Gly
   225                 230                 235                 240
   ggc gga tca agc aac ggc aac ggc aat gtt tgc cct ccc ggc ctc ttc         768
   Gly Gly Ser Ser Asn Gly Asn Gly Asn Val Cys Pro Pro Gly Leu Phe
                   245                 250                 255
   agc aac ccc cag tgc tgt gcc acc caa gtc ctt ggc ctc atc ggc ctt         816
   Ser Asn Pro Gln Cys Cys Ala Thr Gln Val Leu Gly Leu Ile Gly Leu
               260                 265                 270
   gac tgc aaa gtc ccc tcc cag aac gtt tac gac ggc acc gac ttc cgc         864
   Asp Cys Lys Val Pro Ser Gln Asn Val Tyr Asp Gly Thr Asp Phe Arg
           275                 280                 285
   aac gtc tgc gcc aaa acc ggc gcc cag cct ctc tgc tgc gtg gcc ccc         912
   Asn Val Cys Ala Lys Thr Gly Ala Gln Pro Leu Cys Cys Val Ala Pro
       290                 295                 300
   gtt gcc ggc cag gct ctt ctg tgc cag acc gcc gtc ggt gct gga ggc         960
   Val Ala Gly Gln Ala Leu Leu Cys Gln Thr Ala Val Gly Ala Gly Gly
   305                 310                 315                 320
   ggt ggg tct gga ggc ggc gga tca ctg ggt ggc tta ctg ttg ctg tta       1008
   Gly Gly Ser Gly Gly Gly Gly Ser Leu Gly Gly Leu Leu Leu Leu Leu
                   325                 330                 335
   gtg gtt ctg gtt gtg ctg tta gcg acg ccg gtg ctg gtt atc ttc agc       1056
   Val Val Leu Val Val Leu Leu Ala Thr Pro Val Leu Val Ile Phe Ser
               340                 345                 350
   ccg gtt ctg gtc ccg gca gcg ctg gtg gtt ctg ctg tta gtg ctg ttg       1104
   Pro Val Leu Val Pro Ala Ala Leu Val Val Leu Leu Leu Val Leu Leu
           355                 360                 365
   ctg ctg ttg ggt ggc ctg ctc gag ctc gag cac cac cac cac cac cac       1152
   Leu Leu Leu Gly Gly Leu Leu Glu Leu Glu His His His His His His
       370                 375                 380
<210> 9
   <211> 384
   <212> PRT
   <213> Artificial Sequence
<220>
   <223> Fusion protein of class II hydrophobin, artifical polypeptide ArtOLEO and turbo red fluorescent protein (tRFP)
<400> 9
   Met Ala Ser Ser Glu Leu Ile Lys Glu Asn Met His Met Lys Leu Tyr
   1               5                   10                  15
   Met Glu Gly Thr Val Asn Asn His His Phe Lys Cys Thr Ser Glu Gly
               20                  25                  30
   Glu Gly Lys Pro Tyr Glu Gly Thr Gln Thr Met Lys Ile Lys Val Val
           35                  40                  45
   Glu Gly Gly Pro Leu Pro Phe Ala Phe Asp Ile Leu Ala Thr Ser Phe
       50                  55                  60
   Met Tyr Gly Ser Lys Ala Phe Ile Asn His Thr Gln Gly Ile Pro Asp
   65                  70                  75                  80
   Phe Phe Lys Gln Ser Phe Pro Glu Gly Phe Thr Trp Glu Arg Ile Thr
                   85                  90                  95
   Thr Tyr Glu Asp Gly Gly Val Leu Thr Ala Thr Gln Asp Thr Ser Phe
               100                 105                 110
   Gln Asn Gly Cys Ile Ile Tyr Asn Val Lys Ile Asn Gly Val Asn Phe
           115                 120                 125
   Pro Ser Asn Gly Pro Val Met Gln Lys Lys Thr Arg Gly Trp Glu Ala
       130                 135                 140
   Asn Thr Glu Met Leu Tyr Pro Ala Asp Gly Gly Leu Arg Gly His Ser
   145                 150                 155                 160
   Gln Met Ala Leu Lys Leu Val Gly Gly Gly Tyr Leu His Cys Ser Phe
                   165                 170                 175
   Lys Thr Thr Tyr Arg Ser Lys Lys Pro Ala Lys Asn Leu Lys Met Pro
               180                 185                 190
   Gly Phe His Phe Val Asp His Arg Leu Glu Arg Ile Lys Glu Ala Asp
           195                 200                 205
   Lys Glu Thr Tyr Val Glu Gln His Glu Met Ala Val Ala Lys Tyr Cys
       210                 215                 220
   Asp Leu Pro Ser Lys Leu Gly His Arg Gly Gly Gly Gly Ser Gly Gly
   225                 230                 235                 240
   Gly Gly Ser Ser Asn Gly Asn Gly Asn Val Cys Pro Pro Gly Leu Phe
                   245                 250                 255
   Ser Asn Pro Gln Cys Cys Ala Thr Gln Val Leu Gly Leu Ile Gly Leu
               260                 265                 270
   Asp Cys Lys Val Pro Ser Gln Asn Val Tyr Asp Gly Thr Asp Phe Arg
           275                 280                 285
   Asn Val Cys Ala Lys Thr Gly Ala Gln Pro Leu Cys Cys Val Ala Pro
       290                 295                 300
   Val Ala Gly Gln Ala Leu Leu Cys Gln Thr Ala Val Gly Ala Gly Gly
   305                 310                 315                 320
   Gly Gly Ser Gly Gly Gly Gly Ser Leu Gly Gly Leu Leu Leu Leu Leu
                   325                 330                 335
   Val Val Leu Val Val Leu Leu Ala Thr Pro Val Leu Val Ile Phe Ser
               340                 345                 350
   Pro Val Leu Val Pro Ala Ala Leu Val Val Leu Leu Leu Val Leu Leu
           355                 360                 365
   Leu Leu Leu Gly Gly Leu Leu Glu Leu Glu His His His His His His
       370                 375                 380
<210> 10
   <211> 822
   <212> DNA
   <213> Artificial Sequence
<220>
   <223> Gene encoding fusion protein of class II hydrophobin HFBI of Trichoderma reesei and Luciferase from Gaussia princeps
<220>
   <221> CDS
   <222> (1)..(822)
<400> 10
   atg ggc agc agc cat cat cat cat cat cac agc agc ggc ctg gtg ccg       48
   Met Gly Ser Ser His His His His His His Ser Ser Gly Leu Val Pro
   1               5                   10                  15
   cgc ggc agc cat atg agc aac ggc aac ggc aat gtt tgc cct ccc ggc       96
   Arg Gly Ser His Met Ser Asn Gly Asn Gly Asn Val Cys Pro Pro Gly
               20                  25                  30
   ctc ttc agc aac ccc cag tgc tgt gcc acc caa gtc ctt ggc ctc atc      144
   Leu Phe Ser Asn Pro Gln Cys Cys Ala Thr Gln Val Leu Gly Leu Ile
           35                  40                  45
   ggc ctt gac tgc aaa gtc ccc tcc cag aac gtt tac gac ggc acc gac      192
   Gly Leu Asp Cys Lys Val Pro Ser Gln Asn Val Tyr Asp Gly Thr Asp
       50                  55                  60
   ttc cgc aac gtc tgc gcc aaa acc ggc gcc cag cct ctc tgc tgc gtg      240
   Phe Arg Asn Val Cys Ala Lys Thr Gly Ala Gln Pro Leu Cys Cys Val
   65                  70                  75                  80
   gcc ccc gtt gcc ggc cag gct ctt ctg tgc cag acc gcc gtc ggt gct      288
   Ala Pro Val Ala Gly Gln Ala Leu Leu Cys Gln Thr Ala Val Gly Ala
                   85                  90                  95
   gga ggc ggt ggg tct gga ggc ggc gga tca aag ccc acc gag aac aac      336
   Gly Gly Gly Gly Ser Gly Gly Gly Gly Ser Lys Pro Thr Glu Asn Asn
               100                 105                 110
   gaa gac ttc aac atc gtg gcc gtg gcc agc aac ttc gcg acc acg gat      384
   Glu Asp Phe Asn Ile Val Ala Val Ala Ser Asn Phe Ala Thr Thr Asp
           115                 120                 125
   ctc gat gct gat cgc ggg aag ttg ccc ggc aag aag ctg ccg ctg gag      432
   Leu Asp Ala Asp Arg Gly Lys Leu Pro Gly Lys Lys Leu Pro Leu Glu
       130                 135                 140
   gtg ctc aaa gag atg gaa gcc aat gcc cgg aaa gct ggc tgc acc agg      480
   Val Leu Lys Glu Met Glu Ala Asn Ala Arg Lys Ala Gly Cys Thr Arg
   145                 150                 155                 160
   ggc tgt ctg atc tgc ctg tcc cac atc aag tgc acg ccc aag atg aag      528
   Gly Cys Leu Ile Cys Leu Ser His Ile Lys Cys Thr Pro Lys Met Lys
                   165                 170                 175
   aag ttc atc cca gga cgc tgc cac acc tac gaa ggc gac aaa gag tcc      576
   Lys Phe Ile Pro Gly Arg Cys His Thr Tyr Glu Gly Asp Lys Glu Ser
               180                 185                 190
   gca cag ggc ggc ata ggc gag gcg atc gtc gac att cct gag att cct      624
   Ala Gln Gly Gly Ile Gly Glu Ala Ile Val Asp Ile Pro Glu Ile Pro
           195                 200                 205
   ggg ttc aag gac ttg gag ccc atg gag cag ttc atc gca cag gtc gat        672
   Gly Phe Lys Asp Leu Glu Pro Met Glu Gln Phe Ile Ala Gln Val Asp
       210                 215                 220
   ctg tgt gtg gac tgc aca act ggc tgc ctc aaa ggg ctt gcc aac gtg        720
   Leu Cys Val Asp Cys Thr Thr Gly Cys Leu Lys Gly Leu Ala Asn Val
   225                 230                 235                 240
   cag tgt tct gac ctg ctc aag aag tgg ctg ccg caa cgc tgt gcg acc        768
   Gln Cys Ser Asp Leu Leu Lys Lys Trp Leu Pro Gln Arg Cys Ala Thr
                   245                 250                 255
   ttt gcc agc aag atc cag ggc cag gtg gac aag atc aag ggg gcc ggt        816
   Phe Ala Ser Lys Ile Gln Gly Gln Val Asp Lys Ile Lys Gly Ala Gly
               260                 265                 270
   ggt gac                                                                822
   Gly Asp
<210> 11
   <211> 274
   <212> PRT
   <213> Artificial Sequence
<220>
   <223> Fusion protein of class II hydrophobin HFBI of Trichoderma reesei and Luciferase from Gaussia princeps
<400> 11
   Met Gly Ser Ser His His His His His His Ser Ser Gly Leu Val Pro
   1               5                   10                  15
   Arg Gly Ser His Met Ser Asn Gly Asn Gly Asn Val Cys Pro Pro Gly
               20                  25                  30
   Leu Phe Ser Asn Pro Gln Cys Cys Ala Thr Gln Val Leu Gly Leu Ile
           35                  40                  45
   Gly Leu Asp Cys Lys Val Pro Ser Gln Asn Val Tyr Asp Gly Thr Asp
       50                  55                  60
   Phe Arg Asn Val Cys Ala Lys Thr Gly Ala Gln Pro Leu Cys Cys Val
   65                  70                  75                  80
   Ala Pro Val Ala Gly Gln Ala Leu Leu Cys Gln Thr Ala Val Gly Ala
                   85                  90                  95
   Gly Gly Gly Gly Ser Gly Gly Gly Gly Ser Lys Pro Thr Glu Asn Asn
               100                 105                 110
   Glu Asp Phe Asn Ile Val Ala Val Ala Ser Asn Phe Ala Thr Thr Asp
           115                 120                 125
   Leu Asp Ala Asp Arg Gly Lys Leu Pro Gly Lys Lys Leu Pro Leu Glu
       130                 135                 140
   Val Leu Lys Glu Met Glu Ala Asn Ala Arg Lys Ala Gly Cys Thr Arg
   145                 150                 155                 160
   Gly Cys Leu Ile Cys Leu Ser His Ile Lys Cys Thr Pro Lys Met Lys
                   165                 170                 175
   Lys Phe Ile Pro Gly Arg Cys His Thr Tyr Glu Gly Asp Lys Glu Ser
               180                 185                 190
   Ala Gln Gly Gly Ile Gly Glu Ala Ile Val Asp Ile Pro Glu Ile Pro
           195                 200                 205
   Gly Phe Lys Asp Leu Glu Pro Met Glu Gln Phe Ile Ala Gln Val Asp
       210                 215                 220
   Leu Cys Val Asp Cys Thr Thr Gly Cys Leu Lys Gly Leu Ala Asn Val
   225                 230                 235                 240
   Gln Cys Ser Asp Leu Leu Lys Lys Trp Leu Pro Gln Arg Cys Ala Thr
                   245                 250                 255
   Phe Ala Ser Lys Ile Gln Gly Gln Val Asp Lys Ile Lys Gly Ala Gly
               260                 265                 270
   Gly Asp
<210> 12
   <211> 1419
   <212> DNA
   <213> Artificial Sequence
<220>
   <223> Gene encoding fusion protein of core Streptavidin, class I
   hydrophobin CCg2 from Neurosporacrassa and enhanced green fluorescent protein (eGFP)
<220>
   <221> CDS
   <222> (1)..(1419)
<400> 12
   atg ggc agc agc cat cat cat cat cat cac agc agc ggc ctg gtg ccg        48
   Met Gly Ser Ser His His His His His His Ser Ser Gly Leu Val Pro
   1               5                   10                  15
   cgc ggc agc cat atg ggc atc acc ggc acc tgg tac aac cag ctc ggc      96
   Arg Gly Ser His Met Gly Ile Thr Gly Thr Trp Tyr Asn Gln Leu Gly
               20                  25                  30
   tcg acc ttc atc gtg acc gcg ggc gcc gac ggc gcc ctg acc gga acc      144
   Ser Thr Phe Ile Val Thr Ala Gly Ala Asp Gly Ala Leu Thr Gly Thr
           35                  40                  45
   tac gag tcg gcc gtc ggc aac gcc gag agc cgc tac gtc ctg acc ggt      192
   Tyr Glu Ser Ala Val Gly Asn Ala Glu Ser Arg Tyr Val Leu Thr Gly
       50                  55                  60
   cgt tac gac agc gcc ccg gcc acc gac ggc agc ggc acc gcc ctc ggt      240
   Arg Tyr Asp Ser Ala Pro Ala Thr Asp Gly Ser Gly Thr Ala Leu Gly
   65                  70                  75                  80
   tgg acg gtg gcc tgg aag aat aac tac cgc aac gcc cac tcc gcg acc      288
   Trp Thr Val Ala Trp Lys Asn Asn Tyr Arg Asn Ala His Ser Ala Thr
                   85                  90                  95
   acg tgg agc ggc cag tac gtc ggc ggc gcc gag gcg agg atc aac acc      336
   Thr Trp Ser Gly Gln Tyr Val Gly Gly Ala Glu Ala Arg Ile Asn Thr
               100                 105                 110
   cag tgg ctg ctg acc tcc ggc acc acc gag gcc aac gcc tgg aag tcc      384
   Gln Trp Leu Leu Thr Ser Gly Thr Thr Glu Ala Asn Ala Trp Lys Ser
           115                 120                 125
   acg ctg gtc ggc cac gac acc ttc acc aag gtg gga ggc ggt ggg tct      432
   Thr Leu Val Gly His Asp Thr Phe Thr Lys Val Gly Gly Gly Gly Ser
       130                 135                 140
   gga ggc ggc gga tca agc aac ggc aac ggc aat gtt tgc cct ccc ggc      480
   Gly Gly Gly Gly Ser Ser Asn Gly Asn Gly Asn Val Cys Pro Pro Gly
   145                 150                 155                 160
   ctc ttc agc aac ccc cag tgc tgt gcc acc caa gtc ctt ggc ctc atc      528
   Leu Phe Ser Asn Pro Gln Cys Cys Ala Thr Gln Val Leu Gly Leu Ile
                   165                 170                 175
   ggc ctt gac tgc aaa gtc ccc tcc cag aac gtt tac gac ggc acc gac      576
   Gly Leu Asp Cys Lys Val Pro Ser Gln Asn Val Tyr Asp Gly Thr Asp
               180                 185                 190
   ttc cgc aac gtc tgc gcc aaa acc ggc gcc cag cct ctc tgc tgc gtg      624
   Phe Arg Asn Val Cys Ala Lys Thr Gly Ala Gln Pro Leu Cys Cys Val
           195                 200                 205
   gcc ccc gtt gcc ggc cag gct ctt ctg tgc cag acc gcc gtc ggt gct      672
   Ala Pro Val Ala Gly Gln Ala Leu Leu Cys Gln Thr Ala Val Gly Ala
       210                 215                 220
   gga ggc ggt ggg tct gga ggc ggc gga tca atg gtg agc aag ggc gag      720
   Gly Gly Gly Gly Ser Gly Gly Gly Gly Ser Met Val Ser Lys Gly Glu
   225                 230                 235                 240
   gag ctg ttc acc ggg gtg gtg ccc atc ctg gtc gag ctg gac ggc gac      768
   Glu Leu Phe Thr Gly Val Val Pro Ile Leu Val Glu Leu Asp Gly Asp
                   245                 250                 255
   gta aac ggc cac aag ttc agc gtg tcc ggc gag ggc gag ggc gat gcc      816
   Val Asn Gly His Lys Phe Ser Val Ser Gly Glu Gly Glu Gly Asp Ala
               260                 265                 270
   acc tac ggc aag ctg acc ctg aag ttc atc tgc acc acc ggc aag ctg        864
   Thr Tyr Gly Lys Leu Thr Leu Lys Phe Ile Cys Thr Thr Gly Lys Leu
           275                 280                 285
   ccc gtg ccc tgg ccc acc ctc gtg acc acc ctg acc tac ggc gtg cag        912
   Pro Val Pro Trp Pro Thr Leu Val Thr Thr Leu Thr Tyr Gly Val Gln
       290                 295                 300
   tgc ttc agc cgc tac ccc gac cac atg aag cag cac gac ttc ttc aag        960
   Cys Phe Ser Arg Tyr Pro Asp His Met Lys Gln His Asp Phe Phe Lys
   305                 310                 315                 320
   tcc gcc atg ccc gaa ggc tac gtc cag gag cgc acc atc ttc ttc aag       1008
   Ser Ala Met Pro Glu Gly Tyr Val Gln Glu Arg Thr Ile Phe Phe Lys
                   325                 330                 335
   gac gac ggc aac tac aag acc cgc gcc gag gtg aag ttc gag ggc gac       1056
   Asp Asp Gly Asn Tyr Lys Thr Arg Ala Glu Val Lys Phe Glu Gly Asp
               340                 345                 350
   acc ctg gtg aac cgc atc gag ctg aag ggc atc gac ttc aag gag gac       1104
   Thr Leu Val Asn Arg Ile Glu Leu Lys Gly Ile Asp Phe Lys Glu Asp
           355                 360                 365
   ggc aac atc ctg ggg cac aag ctg gag tac aac tac aac agc cac aac       1152
   Gly Asn Ile Leu Gly His Lys Leu Glu Tyr Asn Tyr Asn Ser His Asn
       370                 375                 380
   gtc tat atc atg gcc gac aag cag aag aac ggc atc aag gtg aac ttc       1200
   Val Tyr Ile Met Ala Asp Lys Gln Lys Asn Gly Ile Lys Val Asn Phe
   385                 390                 395                 400
   aag atc cgc cac aac atc gag gac ggc agc gtg cag ctc gcc gac cac       1248
   Lys Ile Arg His Asn Ile Glu Asp Gly Ser Val Gln Leu Ala Asp His
                   405                 410                 415
   tac cag cag aac acc ccc atc ggc gac ggc ccc gtg ctg ctg ccc gac       1296
   Tyr Gln Gln Asn Thr Pro Ile Gly Asp Gly Pro Val Leu Leu Pro Asp
               420                 425                 430
   aac cac tac ctg agc acc cag tcc gcc ctg agc aaa gac ccc aac gag       1344
   Asn His Tyr Leu Ser Thr Gln Ser Ala Leu Ser Lys Asp Pro Asn Glu
           435                 440                 445
   aag cgc gat cac atg gtc ctg ctg gag ttc gtg acc gcc gcc ggg atc       1392
   Lys Arg Asp His Met Val Leu Leu Glu Phe Val Thr Ala Ala Gly Ile
       450                 455                 460
   act ctc ggc atg gac gag ctg tac aag                                   1419
   Thr Leu Gly Met Asp Glu Leu Tyr Lys
   465                 470
<210> 13
   <211> 473
   <212> PRT
   <213> Artificial Sequence
<220>
   <223> Fusion protein of core Streptavidin, class I hydrophobin CCg2 from
   Neurosporacrassa and enhanced green fluorescent protein (eGFP)
<400> 13
   Met Gly Ser Ser His His His His His His Ser Ser Gly Leu Val Pro
   1               5                   10                  15
   Arg Gly Ser His Met Gly Ile Thr Gly Thr Trp Tyr Asn Gln Leu Gly
               20                  25                  30
   Ser Thr Phe Ile Val Thr Ala Gly Ala Asp Gly Ala Leu Thr Gly Thr
           35                  40                  45
   Tyr Glu Ser Ala Val Gly Asn Ala Glu Ser Arg Tyr Val Leu Thr Gly
       50                  55                  60
   Arg Tyr Asp Ser Ala Pro Ala Thr Asp Gly Ser Gly Thr Ala Leu Gly
   65                  70                  75                  80
   Trp Thr Val Ala Trp Lys Asn Asn Tyr Arg Asn Ala His Ser Ala Thr
                   85                  90                  95
   Thr Trp Ser Gly Gln Tyr Val Gly Gly Ala Glu Ala Arg Ile Asn Thr
               100                 105                 110
   Gln Trp Leu Leu Thr Ser Gly Thr Thr Glu Ala Asn Ala Trp Lys Ser
           115                 120                 125
   Thr Leu Val Gly His Asp Thr Phe Thr Lys Val Gly Gly Gly Gly Ser
       130                 135                 140
   Gly Gly Gly Gly Ser Ser Asn Gly Asn Gly Asn Val Cys Pro Pro Gly
   145                 150                 155                 160
   Leu Phe Ser Asn Pro Gln Cys Cys Ala Thr Gln Val Leu Gly Leu Ile
                   165                 170                 175
   Gly Leu Asp Cys Lys Val Pro Ser Gln Asn Val Tyr Asp Gly Thr Asp
               180                 185                 190
   Phe Arg Asn Val Cys Ala Lys Thr Gly Ala Gln Pro Leu Cys Cys Val
           195                 200                 205
   Ala Pro Val Ala Gly Gln Ala Leu Leu Cys Gln Thr Ala Val Gly Ala
       210                 215                 220
   Gly Gly Gly Gly Ser Gly Gly Gly Gly Ser Met Val Ser Lys Gly Glu
   225                 230                 235                 240
   Glu Leu Phe Thr Gly Val Val Pro Ile Leu Val Glu Leu Asp Gly Asp
                   245                 250                 255
   Val Asn Gly His Lys Phe Ser Val Ser Gly Glu Gly Glu Gly Asp Ala
               260                 265                 270
   Thr Tyr Gly Lys Leu Thr Leu Lys Phe Ile Cys Thr Thr Gly Lys Leu
           275                 280                 285
   Pro Val Pro Trp Pro Thr Leu Val Thr Thr Leu Thr Tyr Gly Val Gln
       290                 295                 300
   Cys Phe Ser Arg Tyr Pro Asp His Met Lys Gln His Asp Phe Phe Lys
   305                 310                 315                 320
   Ser Ala Met Pro Glu Gly Tyr Val Gln Glu Arg Thr Ile Phe Phe Lys
                   325                 330                 335
   Asp Asp Gly Asn Tyr Lys Thr Arg Ala Glu Val Lys Phe Glu Gly Asp
               340                 345                 350
   Thr Leu Val Asn Arg Ile Glu Leu Lys Gly Ile Asp Phe Lys Glu Asp
           355                 360                 365
   Gly Asn Ile Leu Gly His Lys Leu Glu Tyr Asn Tyr Asn Ser His Asn
       370                 375                 380
   Val Tyr Ile Met Ala Asp Lys Gln Lys Asn Gly Ile Lys Val Asn Phe
   385                 390                 395                 400
   Lys Ile Arg His Asn Ile Glu Asp Gly Ser Val Gln Leu Ala Asp His
                   405                 410                 415
   Tyr Gln Gln Asn Thr Pro Ile Gly Asp Gly Pro Val Leu Leu Pro Asp
               420                 425                 430
   Asn His Tyr Leu Ser Thr Gln Ser Ala Leu Ser Lys Asp Pro Asn Glu
           435                 440                 445
   Lys Arg Asp His Met Val Leu Leu Glu Phe Val Thr Ala Ala Gly Ile
       450                 455                 460
   Thr Leu Gly Met Asp Glu Leu Tyr Lys
   465                 470
<210> 14
   <211> 1290
   <212> DNA
   <213> Artificial Sequence
<220>
   <223> Gene encoding fusion protein of artifical polypeptide ArtOLEO
   and leucine dehydrogenase
<220>
   <221> CDS
   <222> (1)..(1290)
<400> 14
   atg gaa ctg ttt cgt tat atg gaa cag tat gat tac gaa cag ctg gtg          48
   Met Glu Leu Phe Arg Tyr Met Glu Gln Tyr Asp Tyr Glu Gln Leu Val
   1               5                   10                  15
   ttc tgc cag gat aaa cag tct ggt ctg aaa gcg att atc gcc att cat          96
   Phe Cys Gln Asp Lys Gln Ser Gly Leu Lys Ala Ile Ile Ala Ile His
               20                  25                  30
   gat acc acc ctg ggt ccg gcc ctg ggc ggt acc cgc atg tgg acg tat         144
   Asp Thr Thr Leu Gly Pro Ala Leu Gly Gly Thr Arg Met Trp Thr Tyr
           35                  40                  45
   gaa agt gaa gaa gcg gcc atc gaa gat gca ctg cgt ctg gcg cgc ggt         192
   Glu Ser Glu Glu Ala Ala Ile Glu Asp Ala Leu Arg Leu Ala Arg Gly
       50                  55                  60
   atg acc tac aaa aat gca gcc gcg ggt ctg aat ctg ggc ggt ggc aaa         240
   Met Thr Tyr Lys Asn Ala Ala Ala Gly Leu Asn Leu Gly Gly Gly Lys
   65                  70                  75                  80
   acc gtg att atc ggt gat ccg cgt aaa gat aaa aac gaa gaa atg ttt         288
   Thr Val Ile Ile Gly Asp Pro Arg Lys Asp Lys Asn Glu Glu Met Phe
                   85                  90                  95
   cgt gcg ttc ggc cgc tat att cag ggt ctg aat ggc cgc tac atc acc         336
   Arg Ala Phe Gly Arg Tyr Ile Gln Gly Leu Asn Gly Arg Tyr Ile Thr
               100                 105                 110
   gcc gaa gat gtg ggt acc acg gtt gaa gat atg gat att atc cac gat         384
   Ala Glu Asp Val Gly Thr Thr Val Glu Asp Met Asp Ile Ile His Asp
           115                 120                 125
   gaa acc gat ttt gtt acg ggc att agc ccg gcc ttc ggt agc tct ggc         432
   Glu Thr Asp Phe Val Thr Gly Ile Ser Pro Ala Phe Gly Ser Ser Gly
       130                 135                 140
   aac ccg tct ccg gtg acg gca tat ggt gtt tac aaa ggc atg aaa gca         480
   Asn Pro Ser Pro Val Thr Ala Tyr Gly Val Tyr Lys Gly Met Lys Ala
   145                 150                 155                 160
   gcg gcc aaa gca gcg ttt ggt acc gat agc ctg gaa ggc aaa acg gtg         528
   Ala Ala Lys Ala Ala Phe Gly Thr Asp Ser Leu Glu Gly Lys Thr Val
                   165                 170                 175
   gcg gtt cag ggt gtg ggc aac gtt gcc tat aat ctg tgt cgt cat ctg         576
   Ala Val Gln Gly Val Gly Asn Val Ala Tyr Asn Leu Cys Arg His Leu
               180                 185                 190
   cac gaa gaa ggt gca aaa ctg att gtg acc gat atc aac aaa gaa gcg        624
   His Glu Glu Gly Ala Lys Leu Ile Val Thr Asp Ile Asn Lys Glu Ala
           195                 200                 205
   gtg gaa cgt gca gtt gcg gaa ttc ggc gcc cgc gca gtt gat ccg gat        672
   Val Glu Arg Ala Val Ala Glu Phe Gly Ala Arg Ala Val Asp Pro Asp
       210                 215                 220
   gat att tat tct cag gaa tgc gat atc tac gca ccg tgt gcg ctg ggt        720
   Asp Ile Tyr Ser Gln Glu Cys Asp Ile Tyr Ala Pro Cys Ala Leu Gly
   225                 230                 235                 240
   gcc acc att aat gat gat acg atc ccg cag ctg aaa gcc aaa gtg att        768
   Ala Thr Ile Asn Asp Asp Thr Ile Pro Gln Leu Lys Ala Lys Val Ile
                   245                 250                 255
   gca ggc gcc gca aac aat cag ctg aaa gaa acc cgt cat ggc gat cag        816
   Ala Gly Ala Ala Asn Asn Gln Leu Lys Glu Thr Arg His Gly Asp Gln
               260                 265                 270
   att cac gat atg ggc atc gtg tat gcg ccg gat tac gtt att aac gcc        864
   Ile His Asp Met Gly Ile Val Tyr Ala Pro Asp Tyr Val Ile Asn Ala
           275                 280                 285
   ggt ggc gtg atc aat gtt gca gat gaa ctg tat ggt tac aac agt gaa        912
   Gly Gly Val Ile Asn Val Ala Asp Glu Leu Tyr Gly Tyr Asn Ser Glu
       290                 295                 300
   cgt gcg ctg aaa aaa gtg gaa ggt att tat ggc aat atc gaa cgc gtt        960
   Arg Ala Leu Lys Lys Val Glu Gly Ile Tyr Gly Asn Ile Glu Arg Val
   305                 310                 315                 320
   ctg gaa att agc aaa cgt gat cgc atc ccg acc tac ctg gcc gcg gat       1008
   Leu Glu Ile Ser Lys Arg Asp Arg Ile Pro Thr Tyr Leu Ala Ala Asp
                   325                 330                 335
   cgt ctg gcc gaa gaa cgc att gaa cgt atg cgc cag agt cgt agc cag       1056
   Arg Leu Ala Glu Glu Arg Ile Glu Arg Met Arg Gln Ser Arg Ser Gln
               340                 345                 350
   ttt ctg cag aac ggc cat cac atc ctg tct cgt cgc gga ggc ggt ggg       1104
   Phe Leu Gln Asn Gly His His Ile Leu Ser Arg Arg Gly Gly Gly Gly
           355                 360                 365
   tct gga ggc ggc gga tca ctg ggt ggc tta ctg ttg ctg tta gtg gtt       1152
   Ser Gly Gly Gly Gly Ser Leu Gly Gly Leu Leu Leu Leu Leu Val Val
       370                 375                 380
   ctg gtt gtg ctg tta gcg acg ccg gtg ctg gtt atc ttc agc ccg gtt       1200
   Leu Val Val Leu Leu Ala Thr Pro Val Leu Val Ile Phe Ser Pro Val
   385                 390                 395                 400
   ctg gtc ccg gca gcg ctg gtg gtt ctg ctg tta gtg ctg ttg ctg ctg       1248
   Leu Val Pro Ala Ala Leu Val Val Leu Leu Leu Val Leu Leu Leu Leu
                   405                 410                 415
   ttg ggt ggc ctg ctc gag ctc gag cac cac cac cac cac cac               1290
   Leu Gly Gly Leu Leu Glu Leu Glu His His His His His His
               420                 425                 430
<210> 15
   <211> 430
   <212> PRT
   <213> Artificial Sequence
<220>
   <223> Fusion protein of artifical polypeptide ArtOLEO
   and leucine dehydrogenase
<400> 15
   Met Glu Leu Phe Arg Tyr Met Glu Gln Tyr Asp Tyr Glu Gln Leu Val
   1               5                   10                  15
   Phe Cys Gln Asp Lys Gln Ser Gly Leu Lys Ala Ile Ile Ala Ile His
               20                  25                  30
   Asp Thr Thr Leu Gly Pro Ala Leu Gly Gly Thr Arg Met Trp Thr Tyr
           35                  40                  45
   Glu Ser Glu Glu Ala Ala Ile Glu Asp Ala Leu Arg Leu Ala Arg Gly
       50                  55                  60
   Met Thr Tyr Lys Asn Ala Ala Ala Gly Leu Asn Leu Gly Gly Gly Lys
   65                  70                  75                  80
   Thr Val Ile Ile Gly Asp Pro Arg Lys Asp Lys Asn Glu Glu Met Phe
                   85                  90                  95
   Arg Ala Phe Gly Arg Tyr Ile Gln Gly Leu Asn Gly Arg Tyr Ile Thr
               100                 105                 110
   Ala Glu Asp Val Gly Thr Thr Val Glu Asp Met Asp Ile Ile His Asp
           115                 120                 125
   Glu Thr Asp Phe Val Thr Gly Ile Ser Pro Ala Phe Gly Ser Ser Gly
       130                 135                 140
   Asn Pro Ser Pro Val Thr Ala Tyr Gly Val Tyr Lys Gly Met Lys Ala
   145                 150                 155                 160
   Ala Ala Lys Ala Ala Phe Gly Thr Asp Ser Leu Glu Gly Lys Thr Val
                   165                 170                 175
   Ala Val Gln Gly Val Gly Asn Val Ala Tyr Asn Leu Cys Arg His Leu
               180                 185                 190
   His Glu Glu Gly Ala Lys Leu Ile Val Thr Asp Ile Asn Lys Glu Ala
           195                 200                 205
   Val Glu Arg Ala Val Ala Glu Phe Gly Ala Arg Ala Val Asp Pro Asp
       210                 215                 220
   Asp Ile Tyr Ser Gln Glu Cys Asp Ile Tyr Ala Pro Cys Ala Leu Gly
   225                 230                 235                 240
   Ala Thr Ile Asn Asp Asp Thr Ile Pro Gln Leu Lys Ala Lys Val Ile
                   245                 250                 255
   Ala Gly Ala Ala Asn Asn Gln Leu Lys Glu Thr Arg His Gly Asp Gln
               260                 265                 270
   Ile His Asp Met Gly Ile Val Tyr Ala Pro Asp Tyr Val Ile Asn Ala
           275                 280                 285
   Gly Gly Val Ile Asn Val Ala Asp Glu Leu Tyr Gly Tyr Asn Ser Glu
       290                 295                 300
   Arg Ala Leu Lys Lys Val Glu Gly Ile Tyr Gly Asn Ile Glu Arg Val
   305                 310                 315                 320
   Leu Glu Ile Ser Lys Arg Asp Arg Ile Pro Thr Tyr Leu Ala Ala Asp
                   325                 330                 335
   Arg Leu Ala Glu Glu Arg Ile Glu Arg Met Arg Gln Ser Arg Ser Gln
               340                 345                 350
   Phe Leu Gln Asn Gly His His Ile Leu Ser Arg Arg Gly Gly Gly Gly
           355                 360                 365
   Ser Gly Gly Gly Gly Ser Leu Gly Gly Leu Leu Leu Leu Leu Val Val
       370                 375                 380
   Leu Val Val Leu Leu Ala Thr Pro Val Leu Val Ile Phe Ser Pro Val
   385                 390                 395                 400
   Leu Val Pro Ala Ala Leu Val Val Leu Leu Leu Val Leu Leu Leu Leu
                   405                 410                 415
   Leu Gly Gly Leu Leu Glu Leu Glu His His His His His His
               420                 425                 430

## Beschreibung

Die Erfindung betrifft ein System zum direkten, strahlungslosen Energietransfer über flüssige Phasengrenzen. Die Erfindung eignet sich insbesondere zur Signalübertragung und Signalumwandlung an Phasengrenzen in flüssig-flüssig-Mehrphasensystemen und damit zur Überwachung und Optimierung von chemischen und biochemischen Prozessen in flüssig-flüssig-Mehrphasensystemen.

Aus ökonomischen Gesichtspunkten laufen etwa 90% aller chemischen Produktionsprozesse (zum größten Teil) heterogen katalysiert ab. Diese Mehrphasensysteme werden unterteilt in Systeme mit zwei Phasen (Gas-Flüssig, Gas-Fest, Flüssig-Flüssig, Flüssig-Fest) und Systeme mit drei Phasen (Gas-Flüssig-Fest, Flüssig-Flüssig-Fest). Mehrphasensysteme sind geeignet, um die Vorteile mehrerer Komponenten zusammenzuführen, wobei an ihren Phasengrenzen Reaktionen ablaufen können. Die Komponenten eines Mehrphasensystems sind dabei nur in einer Phase löslich bzw. stabil, oder entfalten dort ihre höchste Reaktivität. Um eine stringente Separation von mindestens zwei Phasen zu gewährleisten, werden in der Technik vorteilhaft synthetische Tenside genutzt. Dabei bestehen Tenside aus ausgeprägten hydrophilen und hydrophoben Bereichen, die eine Vermittlung zwischen beiden Phasen ermöglichen. Um eine Umsetzung von Edukten in einem Mehrphasensystem zu ermöglichen, müssen die Edukte so nah an eine Phasengrenze diffundieren, dass sie an dieser adsorbieren und letztlich unter Bildung von Produkten verbraucht werden. Mehrphasenreaktionen sind daher immer durch eine Überlagerung von physikalischen Transportvorgängen und chemischer Reaktion gekennzeichnet.

Die Prozessüberwachung durch Sensoren basiert meist auf einer Probenentnahme ("near-line" Verfahren) oder Prozessumgehung ("on-line" Verfahren). Bei "near-line" Verfahren müssen dem System ständig Proben entnommen werden, die nach der Messung verworfen werden. Bei geschlossenen Prozessen oder Prozessen, die auf Kleinstvolumina basieren kann dieses Verfahren nicht oder nur bedingt angewendet werden. Zudem verbieten "near-line" als auch "on-line" Verfahren eine direkte Prozessüberwachung. Der elektromagnetische Signalaustausch, das physikalische Grundprinzip des Förster-Resonanzenergietransfers, bietet die Option der minimalinvasiven und nahezu direkten Prozessüberwachung ("in-line") in einem Mehrphasensystem.

Der Förster-Resonanzenergietransfer (FRET) ist ein physikalischer Prozess der Energieübertragung, der durch einen strahlungslosen Energietransfer zwischen einem angeregten Teilchen (Donor) und einem zweiten anregbaren Teilchen (Akzeptor) charakterisiert ist. Im Gegensatz zu makroskopischen Energieübertragungen basiert dieser dabei nicht auf dem Transfer von Lichtteilchen (Photonen) über Abgabe (Emission) und Aufnahme (Absorption). Da die Intensität eines FRETs maßgeblich vom Abstand beider Teilchen abhängt, bedingen bekannte Anwendungen eine Kolokalisierung des Donor/Akzeptorpaares in derselben Phase. In der Technik dient der FRET daher hauptsächlich als "optisches Nanometermaß" für die Abstandsbestimmung zwischen Fluorophoren (Atome, Moleküle, Halbleiternanopartikel, Proteine, usw.) in einem Einphasensystem.

Kurz *et al.* offenbart eine FRET-basierte Nachweisreaktion an einer Phasengrenze eines flüssig-flüssig-Mehrphasensystems mit einer wässrigen und einer organischen Phase (Lipiddoppelschicht) mittels komplementärer Anbindung von Rh-A20mer (ein Rhodaminmarkiertes Adenin-Oligonukleotid) an den komplementären DNA-Strang LT23mer, der durch zwei lipophile Nukleotide in der Lipiddoppelschicht verankert ist (Kurz *et al.* 2006).

Nicolini *et al.* offenbart die direkte Markierung eines Proteins (N-Ras) mit einem für FRET geeigneten Marker (BODIPY) und einer Lipidgruppe zur Verankerung in einer heterogenen Lipiddoppelschicht, wobei sich das Lipid-markierte Protein bevorzugt an der Grenzfläche der "flüssig-ungeordneten" und "flüssig-geordneten Phase" anordnet (Nicolini *et al.* 2005). Weiterhin offenbart Nicolini *et al.* N-Rh-DPPE (Lissamin rhodamine B) als weiteres Molekül, welches für FRET mit BODIPY geeignet ist.

Oritz *et al.* beschreibt die Interaktion zwischen dem Peptid MAP₄VP3 (erstes Molekül) mit der Membran von Lipid-Vesikeln (Liposomen), wobei das Farbstoff-markierte Phospholipid NBD-PE in der Grenzschicht des Liposoms eingebunden ist und der Anstieg des Resonanzenergietransfers vom Tryptophanrest des Peptids auf den organischen Farbstoff ermittelt wird.

Soekarjo *et al.* beschäftigt sich mit der Thermodynamik des Membraninsertionsprozesses des Membranproteins M13 in eine Lipiddoppelschicht. Zur Bewertung der Membranaffinität von M13 wird der Försterresonanzenergietransfer zwischen den Tryptophan- und Tyrosinresten, die natürlich in M13 enthalten sind, und dem Farbstoff-markierten Phospholipid DPH-PC, welches an die Lipidschicht angebunden ist, genutzt.

Optisch aktive Nanopartikel (NP - die auch als Quantum Dots (QDs) bezeichnet werden) können per Lichtemission Informationen über Materialzustände und Prozesse in ihrer Umgebung liefern. Ihre außergewöhnlichen Eigenschaften, die stark von ihrer chemischen Zusammensetzung und Größe abhängen, prädestinieren sie für die Anwendung in schnellen, nichtinvasiven und berührungsfreien optischen Prüfmethoden. Aufgrund ihres großen Oberflächen-zu-Volumenverhältnisses haben Oberflächenmodifikationen einen signifikanten Effekt auf deren elektronische und optische Eigenschaften, sowie ihren Alterungsprozess. Hocheffiziente optisch aktive Nanopartikel werden nach dem Stand der Technik in organischen Lösungsmitteln synthetisiert, um NP mit sehr enger Größenverteilung und damit mit genau definierten Eigenschaften zu erzeugen. Um eine Anwendung in wässrigen Medien gewährleisten zu können, müssen die hergestellten NP nachträglich in die wässrige Phase überführt werden, was mit einem drastischen Aktivitätsverlust der NP verbunden ist. Dabei nimmt die Emissionsquantenausbeute von transferierten NP durch die Abnahme der Oberflächenpassivierung drastisch ab, die normalerweise vom Liganden übernommen wird, und der damit steigenden Anzahl an Oberflächenfehlstellen, in denen die Excitonen strahlungslos relaxieren können.

Die Anwendung von FRET zwischen Quantum Dots und Fluoreszenz-Farbstoff markierten Proteinen wurde bereits beschrieben. Bisher konnte allerdings nur im wässrigen Medium eine Interaktion zwischen NP und Proteinen dokumentiert werden, wobei kein direkter Energietransfer zwischen optisch aktiven NP und anregbaren Proteinen gemessen wurde, sondern lediglich zwischen NP und proteingekoppelten organischen Farbstoffen (Clapp *et al.,* 2003).

Anregbare Proteine sind biologische Makromoleküle, die durch Interaktion mit Photonen oder Elektronen in einen energetisch angeregten Übergangszustand überführt werden können. In Folge der Relaxation in den energetischen Grundzustand wird die absorbierte Energie an die Umgebung in Form eines Photons (Fluoreszenz) oder Elektrons abgegeben.

Mattoussi *et al.* offenbart Protein-Quantumdot (QD)-Konjugate für FRET Untersuchungen, wobei das Maltose-bindende Protein (MBP) in einer wässrigen Phase an wasserlösliche QDs (d.h. die Oberfläche der QDs ist mit Dihydroliponsäure (DHLA) modifiziert) gebunden ist (Mattoussi *et* al., 2004).

Zur Separierung verschiedener Zellkompartimente, oder zur Vermittlung nicht miteinander mischbarer Verbindungen bedient sich die Natur sogenannter Biotenside. Diese sind analog zu synthetischen Tensiden in ausgeprägt hydrophile und hydrophobe Bereiche unterteilt. Amphiphile Proteine, wie zum Beispiel Hydrophobine und Oleosine gehören zur Klasse der hochmolekularen Biotenside. Sie zeichnen sich durch die Ausbildung von hoch stabilen Beschichtungen aus, die im Gegensatz zu synthetischen Tensiden eine erhöhte Biokompatibilität aufweisen (Zampieri *et al.,* 2010; Chiang *et al.,* 2011).

Hydrophobine sind kleine globuläre, amphiphile Proteine, die sich durch ihre Fähigkeit zur (Selbst-)Assemblierung auszeichnen. Sie werden von Pilzen sekretiert, um ihre Verbreitung zu unterstützen. Aufgrund ihrer charakteristischen Eigenschaften sind sie in der Lage die Benetzbarkeit von Oberflächen zu invertieren. Hydrophobine weisen eine hohe Oberflächenaktivität auf, weshalb sie in der Technik zur biokompatiblen Beschichtung von Oberflächen oder zur Stabilisierung von Öl-in-Wasseremulsionen eingesetzt werden (s. u. a. WO 96/41882 A1, WO2004000880 A1, WO2006103252A2).

Joensuu *et al.* offenbart Hydrophobine, die direkt mit GFP oder einem Enzym als Markierungsprotein fusioniert sind (Joensuu *et al.* 2010). Die Extraktion der Fusionsproteine erfolgt mittels eines wässrigen Tensid-basierten Zweiphasensystems.

Oleosine und auch Caleosine sind kleine Strukturproteine, die man in Pflanzenzellen findet, genauer im Ölkörper, dem Oleosom. Sie werden in den Monolayer des Oleosoms integriert und verhindern das unkontrollierte Aggregieren der einzelnen Lipidtröpfchen. WO2012106751 A1 beschreibt die Verwendung von Oleosinen zur Herstellung von künstlichen Öltröpfchen ("oil bodies").

Aufgabe der Erfindung ist es ein System zum direkten, strahlungslosen Energietransfer über flüssig-flüssig-Phasengrenzen anzugeben, welches sich insbesondere zur Signalübertragung und Signalumwandlung an flüssig-flüssig-Phasengrenzen eignet.

Erfindungsgemäß wird die Aufgabe gelöst durch ein System zum strahlungslosen Energietransfer über eine Phasengrenze eines flüssig-flüssig-Mehrphasensystems, welches eine organische Phase und eine wässrige Phase aufweist, enthaltend:
a) ein erstes Molekül, welches für einen Förster-Resonanz-Energie-Transfer (FRET) geeignet ist und sich in der organischen Phase befindet, und
b) ein amphiphiles Protein, welches eine hydrophile Domäne und eine hydrophobe Domäne aufweist und welches mit einem zweiten Molekül markiert ist, welches für einen Förster-Resonanz-Energie-Transfer mit dem ersten Molekül geeignet ist.

Durch die vorliegende Erfindung wird vorteilhaft ein direkter, strahlungsloser Energietransfer über eine flüssig-flüssig-Phasengrenze zwischen mindestens zwei Fluorophoren, bevorzugt einem optisch aktiven Nanopartikel und einem anregbaren Fusionsprotein, ermöglicht.

Das erste Molekül und das zweite Molekül sind so ausgewählt, dass beide Moleküle ein Donor/Akzeptorpaar bilden, welches einen Förster-Resonanzenergietransfer (FRET) ermöglicht, wobei der Donor und der Akzeptor des Donor/Akzeptorpaares durch eine flüssig-flüssig-Phasengrenze voneinander separiert sind.

Das erfindungsgemäße System kennzeichnet sich durch einen Förster-Resonanz-Energie-Transfer (FRET) über eine flüssig-flüssig-Phasengrenze aus. Für den FRET gelten bekannterweise folgende Bedingungen:
i. das Donor/Akzeptorpaar weist eine spektrale Überlappung im Emissionsspektrum des Donors und des Anregungsspektrums des Akzeptors auf,
ii. der Abstand zwischen Donor und Akzeptor liegt im Bereich des Förster-Radius,
iii. der Donor wird durch eine elektromagnetische Strahlung in einen energetisch angeregten Zustand überführt.

Donor/Akzeptorpaare, welche die Bedingung i. erfüllen und entsprechende Anregungsspektren (Bedingung iii.) sind dem Fachmann bekannt. Bevorzugt enthält die organische Phase optisch aktive Nanopartikel als erstes Molekül, bevorzugt als FRET-Donor. Das zweite Molekül ist erfindungsgemäß ein anregbares Protein und liegt bevorzugt als Fusionsprotein mit dem amphiphilen Protein vor.

Das amphiphile Protein weist eine hydrophile Domäne, die in die wässrige Phase zeigt, und eine hydrophobe Domäne, die in die organische Phase zeigt, auf. Das zweite Molekül ist an die hydrophile Domäne gebunden oder bildet diese. So kann das anregbare Protein als Teil eines Fusionsproteins mit der hydrophoben Domäne die hydrophile Domäne bilden, die über einen Linker mit der hydrophoben Domäne verbunden ist. Die hydrophobe Domäne des amphiphilen Proteins stammt vorzugweise von einem Hydrophobin oder Oleosin.

Dass Donor und Akzeptor einen Abstand im Försterradius (Bedingung ii.) aufweisen, wird erfindungsgemäß dadurch erreicht, dass das zweite Molekül (als Donor oder Akzeptor) durch die Anbindung an das amphiphile Protein an der Phasengrenze fixiert wird. Für den FRET ist dies ausreichend, da das erste Molekül durch die Brown'sche Molekularbewegung in die Nähe des fixierten zweiten Moleküls kommt. Zusätzlich und bevorzugt kann das erste Molekül mit dem amphiphilen Protein in Wechselwirkung treten.

Bevorzugt enthält die organische Phase optisch aktive oder fluoreszierende Nanopartikel (NP), die als Donor oder Akzeptor fungieren können.

Der Akzeptor ist bevorzugt ein anregbares Protein, vorzugsweise ein fluoreszierendes oder biolumineszierendes Protein, und liegt bevorzugt als Fusionsprotein mit dem amphiphilen Protein vor. Das amphiphile Protein weist eine hydrophile Domäne und eine hydrophobe Domäne auf. Das anregbare Protein kann selbst die hydrophile Domäne des amphiphilen Proteins bilden. Das amphiphile Protein befindet sich an der Phasengrenze, die hydrophobe Domäne zeigt in die organische Phase. Die hydrophile Domäne, einschließlich dem anregbaren Protein (bevorzugt eine fluoreszierende oder biolumineszente Protein-Einheit) zeigt in die wässrige Phase.

Das amphiphile Protein dient erfindungsgemäß dazu, das zweite Molekül, d. h. den Donor oder den Akzeptor, in die Nähe der Phasengrenze zu bringen. Das amphiphile Protein, bevorzugt ein Hydrophobin oder Oleosin, ist klein und hat vorzugsweise einen Durchmesser kleiner 10 nm. Dadurch wird der Donor oder Akzeptor auf der Seite der wässrigen Phase in einen geringen Abstand (kleiner Förster-Radius), bevorzugte maximale Distanz von 10 nm zu der flüssig-flüssig-Phasengrenze stabilisiert und so ein Förster-Energie-Transfer (FRET) zwischen dem ersten Molekül in der hydrophoben Phase und dem zweiten Molekül an der Phasengrenze ermöglicht. In der organischen Phase befinden sich das erste Molekül, bevorzugt optisch aktive Nanopartikel, frei beweglich und ist aufgrund der Brown'schen Molekularbewegung statistisch in der organischen Phase verteilt. Aufgrund einer Interaktion des ersten Moleküls mit den amphiphilen Proteinen, die an der Öl-Wasserinterphase immobilisiert sind, kommt es zu einer Anreicherung der Nanopartikel an der Phasengrenze (Interphase). Dadurch, dass das erste Molekül, bevorzugt die Nanopartikel, sich bis auf einen gewissen Abstand (R₀) dem amphiphilen Protein annähert, kann ein strahlungsloser Energieübergang zwischen erstem und zweitem Molekül erfolgen.

Zusätzlich kann die hydrophobe Domäne des amphiphilen Proteins eine Bindungsstelle für das erste Molekül, bevorzugt optisch aktive Nanopartikel, aufweisen. Geeignete Bindungsstellen werden vorzugsweise durch Aminosäureresten mit freien Thiol- oder Selenol-Gruppen, bevorzugt durch Cystein- und/oder Selonocystein-Reste, geschaffen.

Die Neuheit der vorgestellten Erfindung liegt in einem FRET, der über eine flüssige Phasengrenze zwischen dem ersten und zweiten Molekül, einem anregbaren Protein und bevorzugt optisch aktiven Nanopartikeln, abläuft.

Bevorzugt umfasst dass amphiphile Protein (ohne Fusionspartner) nur 50 bis 250 Aminosäurereste. Bevorzugte Molekulargewichte liegen zwischen 10 und 30kDa. Bevorzugte Durchmesser liegen zwischen 1,5 und 10 nm, besonders bevorzugt 1,5 und 5 nm. Der Durchmesser kann prinzipiell auch größer sein. Wichtig ist alleine der Abstand zwischen Molekül 1 und Molekül 2. Dieser kann auch durch die Fusionsposition am amphiphilen Protein gewährleistet werden. Die hydrophobe Proteineinheit des amphiphilen Proteins umfasst bevorzugt nur 5 bis 200 Aminosäurereste, bevorzugt 10 bis 200 Aminosäurereste.

Die flüssig-flüssig-Phasengrenze entsteht durch die Mischung einer organischen Phase mit einer wässrigen Phase und wird gleichzeitig durch das amphiphile Protein, vorzugsweise ein Hydrophobin oder Oleosin, stabilisiert. Die organische Phase und eine wässrigen Phase liegen in einer Dispersion, bevorzugt einer Emulsion vor. Die organische Phase enthält erfindungsgemäß ein organisches, nicht mit Wasser mischbares Lösungsmittel oder Öl. Die wässrige Phase ist bevorzugt ein Puffer, Zellkulturmedium oder isotonische Kochsalzlösung.

Durch die Generierung von kleinen artifiziellen Ölkörperchen als organische Phase können biokompatible Sensorsysteme im Mikrometermaßstab entwickelt werden. Sie dienen zum einen als stabilisierte Transportvehikel von reaktiven Komponenten (z. B. Nanopartikel) und können zugleich als Signaltransducer eingesetzt werden.

Die organische Phase und die wässrige Phase liegen bevorzugt in einer Emulsion oder Dispersion vor. Bevorzugt erhöht das amphiphile Protein zusätzlich auch die strukturelle Integrität der flüssig-flüssig-Phasengrenze und damit die Stabilität der Emulsion oder Dispersion. Eine flüssig-flüssig-Phasengrenze weist definitionsgemäß dann eine erhöhte strukturelle Integrität auf, wenn die beiden flüssigen Phasen (im Ruhezustand) eine scharf voneinander getrennte Grenzlinie bilden.

Das erste Molekül, bevorzugt fluoreszierende Nanopartikel, in der organischen Phase ist bevorzugt der FRET-Donor. In diesem Fall ist das zweite Molekül bevorzugt ein fluoreszierendes Protein, welches bei einem Fluoreszenz-Energie-Transfer als FRET-Akzeptor dient.

Alternativ ist das zweite Molekül der FRET-Donor und das erste Molekül der FRET-Akzeptor. In diesem Fall ist das zweite Molekül bevorzugt ein biolumineszentes Protein, welches in einem Biolumineszenz-Energie-Transfer (BRET) Energie auf das erste Molekül, bevorzugt ebenfalls fluoreszierende Nanopartikel, überträgt. Diese Anordnung weist große Vorteile bei der Anwendung als Verstärkersystem auf.

Die fluoreszierenden Nanopartikel haben den Vorteil, dass sie durch ein relativ breites Wellenlängenspektrum angeregt werden können. Die Wellenlänge der Emission lässt sich vorteilhaft durch Größe und Materialien der Nanopartikel einstellen, sodass fasst das ganze sichtbare Spektrum abgedeckt werden kann.

Bevorzugt werden in der Erfindung optisch aktive NP in der hydrophoben Phase eingesetzt. In der hydrophoben Phase haben sie vorteilhaft eine optimierte und höhere Emissionsquantenausbeute. Die Herstellung der NP erfolgt in der Regel in einer hydrophoben Phase. In der Erfindung müssen die optisch aktiven NP vorteilhaft nicht in die wässrige Phase überführt werden. Dies bezüglich erschließt sich ein weiterer Vorteil des beschriebenen Systems, durch die Phasengrenze müssen keine zusätzlichen Reaktionen an den Nanopartikeln durchgeführt werden und somit bleibt die optimierte und hohe Emissionsquantenausbeute erhalten und kann direkt zum Energietransfer genutzt werden. Dies erhöht auch gleichzeitig die Wirtschaftlichkeit des gesamten Prozesses.

Die optisch aktiven NP werden bevorzugt unter inerten Bedingungen in organischen Lösungsmitteln synthetisiert, um NP mit sehr enger Größenverteilung und damit mit genau definierten Eigenschaften zu erzeugen. Zur Darstellung werden schwerlösliche Metallsalze durch organische Stabilisatoren komplexiert und somit in hochreaktive Präkursoren umgewandelt. Die Trennung von Keimbildung und -wachstum nach der Methode von LaMer stellt dabei die Grundlage zur Synthese von monodispersen NP dar (LAMER *et al.,* 1950). Als Stabilisatoren und Lösungsvermittler werden langkettige Liganden eingesetzt, die einen Kopf-Schwanz-Aufbau aufweisen. Die polare Kopfgruppe dient der direkten Interaktion mit dem Nanopartikel, wohingegen der unpolare Schwanz als Lösungsvermittler im organischen Lösungsmittel wirkt.

Vorteilhaft müssen in der Erfindung die Nanopartikel nicht in die wässrige Phase überführt werden, sondern werden bevorzugt in der organischen Phase eingesetzt, wodurch ihre Aktivität und die Emisionsquantenausbeute deutlich höher sind und optimal genutzt werden.

### Optisch aktive Nanopartikel

Die organische Phase beinhaltet vorzugsweise optisch aktive Nanopartikel als erstes Molekül, die entweder als Donor oder Akzeptor in einem Donor/Akzeptorpaar fungieren können. Besonders bevorzugt sind halbleitende Nanopartikel aus Verbindungen von Metallen oder Halbmetallen ausgewählt aus den Gruppen 8 bis 14, bevorzugt der Gruppen 12 und 14 des Periodensystems der Elemente. Besonders bevorzugt sind Verbindungen von Zn, Cd, Hg, Sn, Pb und/oder In. Als Gegenionen weisen die Metalle oder Halbmetalle bevorzugt Elemente aus den Gruppen 15 und 16 auf, besonders bevorzugt sind O, S, Se und/oder Te. Ideale halbleitende Nanopartikel zeigen eine hohe Emissionsquantenausbeute. Besonders bevorzugt sind Nanopartikel aus Cadmiumsulfid (CdS), Cadmiumselenid (CdSe), Cadmiumquecksilbertelurit (CdHgTe), Indiumarsenid (InAs), Zinksulfid (ZnS) und/oder Zinkselenid (ZnSe). Für eine Erhöhung der Emissionsquantenausbeute, können die halbleitenden Nanopartikel mit einer dünnen Schicht an anorganischem Material (bevorzugt Metallchalkogenide, bspw. CdSe oder ZnS) überzogen sein und/oder mit Elementen dotiert (bevorzugt In, Mn) sein. Die Nanopartikel haben typischerweise einen Durchmesser von 5 bis 50 nm, bevorzugt 1 bis 15 nm und besonders bevorzugt 1 bis 10 nm.

Die wässrige Phase beinhaltet erfindungsgemäß anregbare Proteine, die bevorzugt an das amphiphile Protein fusioniert sind, als zweites Molekül. Dabei ist das anregbare Protein - insbesondere als Akzeptor - bevorzugt ein fluoreszierendes Protein, bevorzugt ein Derivat des grün fluoreszierenden Proteins (GFP) oder des rot fluoreszierenden Proteins (RFP). Alternativ bevorzugt ist das anregbare Protein - insbesondere als Donor - eine Oxigenase, bevorzugt eine Luciferase aus *Gaussia princeps.* Weiter bevorzugt ist oder beinhaltet das anregbare Protein eine Oxidoreduktase, bevorzugt eine Oxidase oder Dehydrogenase, wie bspw. die Leucindehydrogenase.

Als amphiphile Proteine werden bevorzugt Hydrophobine, Chapline, Oleosine, Caleosine, rekombinante und/oder artifizielle Polypeptide eingesetzt, wobei die rekombinanten Polypeptide bevorzugt eine hydrophobe Domäne einer der vorgenannten Proteine enthalten. Bevorzugte Hydrophobine werden unter anderem in US2009104663 und WO2006103252A2 genannt.

Die Positionen der polaren und unpolaren Aminosäurereste in der Hydrophobin-Einheit sind konserviert und umfassen einen charakteristischen hydrophoben Knoten. Für die Definition von Hydrophobinen ist dabei primär die Struktur und nicht die Sequenzspezifität der Hydrophobine entscheidend. Die Aminosäuresequenz der natürlichen Hydrophobine ist sehr divers, sie haben jedoch alle ein hochcharakteristisches Muster von 6, bevorzugt 8, konservierten Cysteinresten. Diese Reste bilden vier intramolekulare Disulfidbrücken. Der N- und C-Terminus ist über einen größeren Bereich variabel. Hier können mittels dem Fachmann bekannten molekularbiologischen Techniken Fusionspartnerproteine mit einer Länge von 10 bis 500 Aminosäuren angefügt werden.

Hydrophobine gehören einer Familie gut charakterisierter Proteinen an und umfassen insbesondere Polypeptide, welche die folgende allgemeine konservierte Strukturformel (I) enthalten

C¹-X₅₋₉-C²-C³-X₁₁₋₃₉C⁴-X₂₋₂₃-C⁵X₅₋₉C⁶-C⁷-X₆₋₁₈-C⁸ (I)

wobei X für jede der 20 proteinogenen Aminosäuren (Ala, Arg, Asn, Asp, Cys, Gin, Glu, Gly, His, Ile, Leu, Lys, Met, Phe, Pro, Ser, Thr, Trp, Tyr, Val) stehen kann. Dabei können die X jeweils gleich oder verschieden sein. Hierbei stellen die bei X stehenden Indizes jeweils die Anzahl der Aminosäuren dar, C steht für Cystein, Alanin, Serin, Glycin, Methionin oder Threonin, wobei mindestens sechs, bevorzugt 7, besonders bevorzugt alle 8, der mit C benannten Reste für Cystein stehen. Die Cysteine liegen entweder reduziert vor oder bilden miteinander Disulfidbrücken aus. Besonders bevorzugt ist die intramolekulare Ausbildung von mindestens zwei, besonders bevorzugt drei, und ganz besonders bevorzugt vier intramolekularen Disulfidbrücken.

Erfindungsgemäß werden bevorzugt rekombinante Hydrophobine eingesetzt, welche die Hydrophobin-Einheit oder zumindest den hydrophoben Knoten oder ein Polypeptid gemäß Formel I umfassen. Dieser bildet die hydrophobe Domäne des amphiphilen Proteins. Hydrophobine sind weiterhin durch die Eigenschaft charakterisiert, dass sie bei Raumtemperatur nach Beschichten einer Glasoberfläche eine Veränderung des Kontaktwinkels eines Wassertropfens von mindestens 20°, bevorzugt mindestens 25° und besonders bevorzugt 30° bewirken, jeweils verglichen mit dem Kontaktwinkel eines gleich großen Wassertropfens mit der unbeschichteten Glasoberfläche.

Vorzugsweise gehören die Hydrophobine der Klasse I an, wie dewA, rodA, hypA, hypB, sc3, basf1, basf2, besonders bevorzugt Ccg2 aus *Neurospora crassa.* Weiter bevorzugt sind Hydrophobine der Klasse II, vorzugsweise HFBI oder HFBII aus *Trichoderma reesei.*

Der Begriff Hydrophobin umfasst auch aus Bakterien-isolierte Hydrophobin-ähnliche Proteine, wie sie in der WO0174864 A1 offenbart werden oder die aus dem *E. Coli* Stamm CBS 102638 hinterlegt am 14.03.2000 im Centraalbureau voor Schimmelcultures (Oosterstraat 1, P. O. Box 273, 3740 AG Baarn, Niederlande) isoliert werden können.

Chapline sind eine Familie von hydrophoben Proteinen aus Bakterien, insbesondere der Gattung Streptomyces. Bevorzugte Chapline sind ausgewählt aus ChpA, ChpB, ChpC, ChpD, ChpE, ChpF, ChpG und ChpH, Bevorzugte Chapline enthalten eine hydrophobe Domäne mit folgender allgemeinen Strukturformel (II):

SPGX₄X₅SGNX₉X₁₀QX₁₂PX₁₄X₁₅X₁₆PVX₁₉X₂₀X₂₁GNX₂₄X₂₅X₂₆VX₂₈X₂₉X₃₀LN**P**AX₃₅GNX₃₈X₃₉X₄₀X₄₁ (II)

wobei
X₄, X₅, X₁₀, X₁₂, X₁₄, X₁₆, X₂₀, X₂₅, X₂₈, X₃₀ unabhängig voneinander ausgewählt sind aus V, L, I und A,
X₉ unabhängig voneinander ausgewählt sind aus V, L, I, T, S und A,
X_{15,} X₁₉ und X₄₁ unabhängig voneinander ausgewählt sind aus H, N und D,
X₂₁unabhängig voneinander ausgewählt sind aus C, V, L, I und A,
X₂₄ ausgewählt ist aus T und S,
X₂₆ ausgewählt ist aus N, D und S,
X₂₉ ausgewählt ist aus G und A,
X₃₅ ausgewählt ist aus A, F und M,
X₃₈ und X₄₀ ausgewählt sind aus beliebigen proteinorgen Aminosäureresten, bevorzugt A, G, V, K, L,I, E, D,
X₃₉ ausgewählt ist aus G und C.

Natürlich vorkommende Oleosine bestehen aus drei Teilen: Die N- und die C-terminale Domäne sind hydrophil, der mittlere Teil hingegen ist hydrophob. Modelle zeigen, dass die mittlere hydrophobe Domäne der Oleosine eine Haarnadel-ähnliche Form aufweist, die in die Triacylglycerid-Schicht hineinragt, während die hydrophilen Domänen außerhalb des Ölkörpers bleiben.

Bevorzugte Oleosine werden unter anderem in der EP1902132 A2 und WO2012106751 A1 genannt.

Erfindungsgemäß werden bevorzugt rekombinante Oleosine eingesetzt, die zumindest die hydrophobe Domäne mit der Haarnadel-ähnlichen Form umfassen. Dieser bildet die hydrophobe Domäne des amphiphilen Proteins und umfasst insbesondere Polypeptide, welche die folgende allgemeine konservierte Strukturformel (III) enthalten,

X₁₋₃₀-P-X₁₋₅-P-S-X₁₋₅-P-X₁₋₃₀ (III)

oder weiter bevorzugt

X₅₋₂₀-P-X₂₋₄-P-S-X₂₋₄-P-X₅₋₂₀ (IV)

wobei der Index jeweils für die Anzahl Aminosäurereste steht und X für jede der 20 natürlichen Aminosäuren (Ala, Arg, Asn, Asp, Cys, Gln, Glu, Gly, His, Ile, Leu, Lys, Met, Phe, Pro, Ser, Thr, Trp, Tyr, Val) stehen kann. Dabei können die X jeweils gleich oder verschieden sein. Mindestens 50%, bevorzugt 70%, der X sind ausgewählt aus den unpolaren Aminosäuren Ala, Gly, Iso, Leu, Met, Phe, Pro, Trp und Val. Hierbei stellen die bei X stehenden Indizes jeweils die Anzahl der Aminosäuren dar. P steht für Prolin und S für Serin.

Die hydrophile Domäne des amphiphilen Proteins kann bevorzugt entweder durch einen amphiphilen Teil des Oleosins oder durch das anregbare Protein gebildet werden oder auch beide umfassen. Die Oleosine unterscheiden sich je nach Spezies in Ihrer Primärsequenz.

Für die Definition von Oleosinen ist ebenfalls die Struktur und nicht die Sequenzspezifität der Oleosine entscheidend.

Bevorzugt sind Oleosine, die in den Samen von Mais (Zea *mays,* besonders bevorzugt NP_001151906.1, NP_001105338.1 oder NP_001152614.1) oder Reis (*Oryza sativa,* besonders bevorzugt NP_001053473 oder NP_001062874) gefunden wurden (VANCE & HUANG *et al.,* 1987).

Caleosine sind den Oleosinen strukturell und funktionell sehr ähnlich und können ebenfalls zum Einsatz kommen. Bevorzugte Caleosine bzw. deren hydrophober Teil enthalten ebenfalls die hydrophobe Domäne mit der Haarnadel-ähnlichen Form gemäß Strukturformel (II) oder vorzugsweise Formel (IV) und sind vorzugsweise ausgewählt aus Sop1, Arab-1 (AC002332), Arab-2 (AF067857), Caleosin aus Reis (X89891) und Sojabohnen (AF004809).

Bei den artifiziellen Polypeptiden handelt es sich um abgeleitete Hydrophobine, Chapline, Caleosine und/oder Oleosine, die auf Grund der Nachbildung der Anordnung von hydrophoben und hydrophilen Aminosäuren bevorzugt zu einer selbstassemblierenden Wirkung befähigt sind. Als hydrophobe Aminosäuren dienen bevorzugt die unpolaren Aminosäuren Ala, Gly, Iso, Leu, Met, Phe, Pro, Trp und Val. Als hydrophile Aminosäure dient bevorzugt Histidin, welche in einem Polypeptid bestehend aus sechs aufeinander folgenden Histidinen angeordnet ist.

Das erfindungsgemäße System eignet sich insbesondere zum Signalaustausch und zur Signalumwandlung eines Fluoreszenzsignals über eine elektromagnetische Kaskade bestehend aus mindestens einem Donor/Akzeptorpaar. Der Akzeptor ist generell dadurch gekennzeichnet, dass er elektromagnetische Strahlung absorbiert und anschließend elektromagnetische Strahlung in einem eng definierten Wellenlängenbereich, vorzugsweise im Bereich umfassend 50-100 nm emittiert. Der Wellenlängenbereich des Emissionsspektrums des finalen Akzeptors ist dabei vorteilhaft zu höheren Wellenlängen im Vergleich zum Emissionsspektrum des Donors verschoben. Diese vorteilhafte Anordnung des mindestens einen Donor/Akzeptorpaares erlaubt die simultane Signalumwandlung von unterschiedlichen Donoremissionsspektren mit einer unveränderlichen Detektoreinstellung.

Indirekt eignet sich das System zur Detektion der Integrität der Phasengrenzen in einem flüssig-flüssig-Mehrphasensystem. Wenn die Phasengrenzen intakt sind, befinden sich die fluoreszierenden Nanopartikel (nahezu) ausschließlich in der organischen Phase. Durch den Kontakt mit dem Fluorophor-fusionierten, amphiphilen Protein entsteht ein FRET über die Phasengrenzen hinweg. Sofern die Phasen nicht intakt sind, kollabiert die Ligandenhülle was zur Agglomeration der Nanopartikel führt. Dadurch wird das Signal abgeschwächt bzw. vollständig gelöscht. Durch die vorliegende Erfindung können biokompatible Prozessabläufe in Mehrphasensystemen überwacht werden. Das erfindungsgemäße System eignet sich zur Anwendung als biokompatibles Sensorsystem im Mikrometermaßstab.

Aufgrund der hohen Biokompatibilität, die durch die amphiphilen Proteine vermittelt wird, sowie der hohen Langzeitstabilität der organischen Phase, die hier bevorzugt als künstlichen Öltropfen vorliegt, liegt ein weiteres Anwendungsgebiet der Erfindung in der medizinischen Anwendung. Dabei kann ein Energietransfer von Nanopartikeln, die in der organischen Phase lokalisiert sind über ein anregbares Protein in der wässrigen Phase auf eine Gewebezelle bzw. auf Zellen von Mikroorganismen, Bakterien oder Parasiten ermöglicht werden.

Durch eine weitere Fusion des Fluorophor-fusionierten amphiphilen Proteins mit einem Antigen oder dessen Epitop kann ein künstlicher Öltropfen, auf dessen Oberfläche ein Antigen oder dessen Epitop exponiert ist, analog zu Antikörper-Antigen-Bindungen an einen Rezeptor auf der Oberfläche von Zellen binden. Nach Anregung der Nanopartikel in der organischen Phase, können diese die absorbierte Energie auf das anregbare Protein übertragen, wobei thermische Energie frei wird. Auf Grund der Nähe zu lebendem Material, kommt es zu einer gezielten lokalen Temperaturerhöhung, die zur Zersetzung des Materials führt.

Eine weitere Anwendung der Erfindung liegt in der Generierung von regenerierbaren enzymatischen Katalysesystemen. Die organische Phase liegt hier bevorzugt als künstliche Öltropfen vor, die mikrostrukturierte Zweiphasensystem darstellen und ein großes Oberflächen-zu-Volumenverhältnis aufweisen. Ein großes Oberflächen-zu-Volumenverhältnis ist vorteilhaft in Prozessen, die kurze Ansprechzeiten, hohe Stoff- oder Hitzetransferraten benötigt. Somit stellen künstliche Öltropfen ein miniaturisiertes Reaktionssystem dar, welches sich durch einen geringen Material- und Energieverbrauch auszeichnet.

In einer bevorzugten Variante des erfindungsgemäßen Systems ist an das amphiphile Protein zusätzlich eine Domäne mit enzymatischer Aktivität gekoppelt. Die Kopplung kann prinzipiell durch Crosslinking geschehen. Bevorzugt ist die Domäne jedoch ein weiterer Fusionspartner. Das amphiphile Protein ist dabei vorteilhaft wie oben beschrieben ausgewählt, welches eine Distanz kleiner 5 nm zwischen dem anregbaren Protein und der flüssig-flüssig-Phasengrenze gewährleistet. Somit befinden sich Donor und Akzeptor in einem Abstand zueinander, der einen Dexterelektronentransfer ermöglicht.

Vorteilhaft dient in dieser Variante das erfindungsgemäße System nicht nur der Detektion sondern auch zur Generierung von enzymatischen Katalysesystemen, die ein großes Oberflächen-zu-Volumenverhältnis aufweisen.

Bevorzugte anregbare Proteine sind in dieser Variante Proteine die Elektronen übertragen können und in biochemische Redoxreaktionen als Elektronenüberträger Reaktionen katalysieren können. Vorteilhafterweise ist das anregbare Protein eine Oxidoreduktase.

In einem alternativen Aufbau kann die Erfindung zum Beispiel zur Zell-freien Wasserstoffgewinnung genutzt werden. Basierend auf Oleosinen oder Größen-optimierten artifiziellen Polypeptiden kann der Abstand zwischen dem Donor und dem Akzeptor unterhalb einer Distanz von 5 nm verkürzt werden. Ein solcher maßgeschneiderter Aufbau ermöglicht die wechselseitige Elektronenübertragung von einer Oxidoreduktase in einem wässrigen Medium auf einen synthetischen Katalysator, der in der organischen Phase lokalisiert ist. In einer ersten Reaktion wird dabei Licht von Nanopartikeln - bevorzugt in der organischen Phase - aufgefangen und konzentriert. Die so gesammelte Strahlungsenergie ermöglicht den Aufbau eines Ladungsgradientens an der flüssig-flüssig-Phasengrenze, wobei ein angeregtes Elektron auf das katalytische Zentrum der Oxidoreduktase transferiert wird. Die sich anschließende Reaktion nutzt die entstandenen positiven Ladungen der Nanopartikel um den Sauerstoff des Wassers zu oxidieren. Die im Reaktionszentrum der Oxidoreduktase verbleibenden Elektronen werden auf Wasserstoff übertragen. Bevorzugte synthetische Katalysatoren sind dabei metallorganische Verbindungen, die als Metallion bevorzugt Ruthenium, Rhodium, Platin oder Palladium enthalten.

Anhand folgender Figuren und Ausführungsbeispiele soll die Erfindung näher erläutert werden, ohne die Erfindung auf diese zu beschränken.

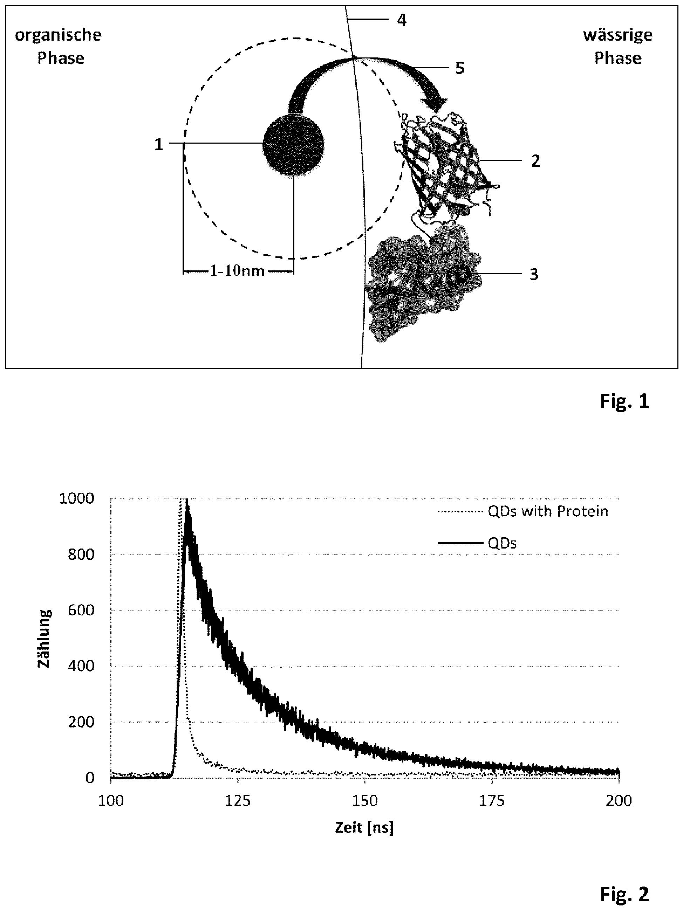

**Fig.** 1 zeigt eine schematische Darstellung des erfindungsgemäßen Fluoreszenzenergietransfers **5** über die Phasengrenzen. Exemplarisch ist der Energietransfer von einem optisch aktiven Nanopartikel **1** in der organischen Phase auf ein anregbares Protein **2** in der wässrigen Phase dargestellt. Für einen effektiven Energietransfer müssen sich die Partikel in einem gewissen Abstand (gestrichelte Linie) zueinander befinden, der durch den Förster-Radius R₀ (bevorzugt zwischen 1 und 10 nm) definiert ist. Das amphiphile Protein **3** sorgt als Mediator für eine nahe Immobilisierung des anregbaren Proteins **2** an der flüssig-flüssig-Phasengrenze **4.**

Wie in Fig. **1** dargestellt ist, erlaubt der Einsatz von amphiphilen Proteinen **3** (bevorzugt Hydrophobin- oder Oleofin-Fusionsproteine) die gezielte Immobilisierung von anregbaren (optisch aktiven) Proteindomänen an einer Wasser-Öl-Interphase **4.** Die grün und rot fluoreszierenden Proteine (eGFP bzw. tRFP) wurden als generelle Modelproteine für anregbare Proteine eingesetzt.

**Fig. 2** zeigt eine Lebenszeitmessung des angeregten Zustands der CdS/ZnS "core-shell"-Nanopartikel.

**Fig. 3** zeigt die zeitabhängige Fluoreszenzintensität des Akzeptors (Ccg2-tRFP). Aufgetragen ist die Fluoreszenzintensität des Akzeptors über der Zeit für unterschiedliche Konzentrationen des Donors (CdSe-Partikel) und gleiche Konzentrationen des Akzeptors (Ccg2-tRFP).

**Fig. 4** zeigt die Sensitivität des FRETs. Mit steigendem Konzentration des Donors (CdSe Partikel) im Verhältnis zum Akzeptor (Ccg2-tRFP), steigt die Fluoreszenzintensität des Akzeptors (Ccg2-tRFP).

### Ausführungsbeispiel1: Expression rekombinanter Fluorophor-fusionierter, amphiphiler Fusionsproteine in E. coli und deren Reinigung.

Es wurden die in Tabelle 1 genannten rekombinanten Fluorophor-fusionierten, amphiphilen Fusionsproteine generiert, welche jeweils eine amphiphile Proteindomäne enthalten und eine darin bezeichnete anregbare Proteindomäne enthalten.

**Tabelle 1:**

| **Fusionsprotein** | **Amphiphile Proteindomäne** | **Anregbare Proteindomäne** | **SEQ ID Nr. DNA** | **SEQ ID Nr. Protein** |
|---|---|---|---|---|
| Ccg2-tRFP | Klasse I Hydrophobin Ccg2 (*Neurospora crassa*) | Turbo rot fluoreszierendes Protein tRFP (abgeleitet vom *Entacmaea quadricolor-*basierenden GFP) | 2 | 3 |
| HFBI-eGFP | Klasse II Hydrophobin HFBI (*Trichoderma reesei*) | Grün fluoreszierendes Protein eGFP (*Aequorea victoria*) | 4 | 5 |
| HFBI-tRFP | Klasse II Hydrophobin HFBI (*Trichoderma reesei*) | Turbo rot fluoreszierendes Protein tRFP (abgeleitet vom *Entacmaea quadricolor-*basierenden GFP) | 6 | 7 |
| tRFP-HFBI-ArtOLEO | Klasse II Hydrophobin HFBI (*Trichoderma reesei*) und artifizielles Polypeptid ArtOLEO | Turbo rot fluoreszierendes Protein tRFP (abgeleitet vom *Entacmaea quadricolor-*basierenden GFP) | 8 | 9 |
| HFBI-GLuc | Klasse II Hydrophobin HFBI (*Trichoderma reesei*) | Luciferase (*Gaussia princeps*) | 10 | 11 |
| cStrep-Ccg2-eGFP | Klasse I Hydrophobin Ccg2 (*Neurospora crassa*) | Grün fluoreszierendes Protein eGFP (*Aequorea victoria*) | 12 | 13 |
| ArtOLEO-LeuDH | artifizielles Polypeptid ArtOLEO | Leucin-Dehydrogenase (*Bacillus lichiformis*) | 14 | 15 |

Dazu wurden die für die jeweilige Domäne kodierenden DNA-Sequenzen mittels Polymerasekettenreaktion (PCR) amplifiziert und über eine overlap PCR wurde ein Fusionsgen zusammengefügt. Die verwendeten flankierenden Primer enthielten Erkennungssequenzen für die Restriktionsendonukleasen *Nde*I und *Xho*I*.* Nach Vektor- und Fragmentrestriktion über die entsprechenden Endonukleasen, Ligation und Transformation in *Escherichia coli* (*E. coli*) als Wirtsorganismus, wurde die Anwesenheit des Fusionsgens in den generierten Plasmiden überprüft.

Für die Produktion des selbstassemblierenden Protein enthaltenden Fusionsproteins, wurden die Plasmide, die das generierte Fusionsgen korrekt enthielten, in den *E. coli* Stamm SHuffle^{®} T7 Express transformiert. Das Fusionsprotein wird in den Bakterienzellen intrazellulär angereichert.

Die transformierten *E. coli* Zellen wurden bei 30°C kultiviert. Die Expression des Fusionsgens wurde durch Zugabe von 0,4 mM (mmol/l) IPTG induziert. Nach 4-6 h wurden die Zellen mittels Zentrifugation (bei 4000 x g, 4°C, 10 min) pelletiert. Nach zweimaligem Waschen des Zellpellets mit 50 mM Trispuffer (pH 7,5) wurden die Zellen erneut durch Zentrifugation pelletiert. Zum Zellaufschluss wurden die *E*. *coli* Zellen einer dreifachen Ultraschallbehandlung (9 cyc., 70%, 4°C, 2 min) und einer anschließenden dreimaligen French Press-Behandlung (20'000 psi) unterzogen. Die aufgeschlossenen Bakterienzellen wurden zweimal mit 50 mM Trispuffer (pH 7,5) gewaschen. Zur Proteinextraktion wurden die aufgeschlossenen Zellen in 1 ml Lysepuffer aufgenommen und bei 23 °C für 30 min inkubiert. Die Reinigung des Fusionsproteins erfolgte mittels Nickelaffinitätschromatographie. Die Elution des Fusionsproteins erfolgte in einem isotyp zusammengesetzten Phosphatpuffer (pH 4,5) mit 250 mM Imidazol.

Um das Protein in einen aktiven Zustand zu überführen, wurde das erhaltene Eluat gegen das 4000fache Volumen eines Dialysepuffers bei 4 °C für maximal 48 h dialysiert. Zur Dialyse von Hydrophobinen wurde ein Tris-Puffer (50 mM, pH 8,5 mit 1 mM Glutathion reduziert und 0,2 mM Glutathion oxidiert) verwendet. Zur Dialyse von Surface-Layer-Proteinen wurde 10 mM Tris-Puffer (pH 9,0) als Dialysepuffer eingesetzt.

### Ausführungsbeispiel 2: Erzeugung einer Protein-stabilisierten Emulsion und konvokale Fluoreszenzmikroskopie.

Es wurde eine Protein-stabilisierte Öl-in-Wasser-Emulsion erzeugt. Dazu wurde das Fusionsprotein Ccg2-tRFP eingesetzt, welches als amphiphile Proteindomäne ein Klasse I Hydrophobin (Ccg2 aus *Neurospora crassa*) enthält und als anregbare Proteindomäne das tRFP (abgeleitet vom *Entacmaea* quadricolor-basierenden GFP). Die organische Phase enthält CdSe-Partikel als optisch anregbare Nanopartikel, die ein Absorptionsmaximum bei 368 nm aufweisen und ein Emissionsmaximum bei 488 nm aufweisen.

Zur Erzeugung einer Protein-stabilisierten Emulsion werden 0,5 *µ*l einer organischen Phase (1-Octadecen, Mineralöl oder Olivenöl) in einem Eppendorfgefäß vorgelegt. Die organische Phase enthält CdSe-Partikel als optisch anregbare Nanopartikel und wird überschichtet mit 180 *µ*l einer wässrigen Proteinlösung, beinhaltend 200 ng/*µ*l des Fusionsproteins Ccg2-tRFP in 50 mM Tris-Puffer (pH 8,5). Nach einer Ultraschallbehandlung (4 Zyklen, 75%, Raumtemperatur) mit einem Bandelin Sonopuls UW 2070 von 20 Sekunden wird die Emulsion mit weiteren 180 *µ*l der wässrigen Proteinlösung gemischt und für 5 Minuten bei Raumtemperatur belassen. Anschließend wird die Mischung erneut einer Ultraschallbehandlung (4 Zyklen, 75%, Raumtemperatur) von 20 Sekunden unterzogen und die Emulsion mit weiteren 180 *µ*l der wässrigen Proteinlösung gemischt.

Die generierten Öltröpfchen wurden mit einem konvokalen ApoTome1 Mikroskop (Carl Zeiss Microlmaging GmbH, Deutschland) und geeigneten Filtern (FS49 DAPI und FS43 DsRed) untersucht. Bei selektiver Anregung der CdSe-Partikel mit dem FS49 DAPI Filtersetup war die Fluoreszenz der Öltröpfchen im blauen Spektrum einheitlich, was auf eine homogene Verteilung der Nanopartikel in der organischen Phase schließen lässt. Nach 3 Stunden wurde eine ausgeprägt blau fluoreszierende "Korona" um die Öltröpfchen detektiert, was auf eine Anreicherung der CdSe-Partikel in der flüssig-flüssig-Phasengrenze hindeutet. Hingegen war bei einer selektiven Anregung des tRFPs mit dem FS43 DsRed Filtersetups über den gesamten Versuchszeitraum ausschließlich eine rote Fluoreszenz an der flüssig-flüssig-Phasengrenze detektierbar. Dies bestätigt die Anhaftung des Fluorophor-fusionierten, amphiphilen Proteins an der flüssig-flüssig-Phasengrenze. Die selektive Anregung der CdSe-Partikel mit einem FS49 DAPI Filtersetup und die simultane Auslesung der Emission des tRFPs mit einem FS43 DsRed Filtersetups bilden deckungsgleiche Mikroskopieaufnahmen ab, wie bei der selektiven Anregung und Auslesung der tRFP-Fluoreszenzsignale mit dem FS43 DsRed Filtersetup.

Die erhaltenen fluoreszenzmikroskopischen Aufnahmen verbildlichen eine direkte Interaktion zwischen den CdSe-Partikeln und dem Fluorophor-fusionierten, amphiphilen Protein.

### Ausführungsbeispiel 3: Lebenszeitmessung.

Es wurde eine Protein-stabilisierte Emulsion analog zu Ausführungsbeispiel 2 mit dem Fluorophor-fusionierten, amphiphilen Protein Ccg2-tRFP generiert. Die organische Phase enthielt CdS/ZnS "core-shell" Partikel als optisch aktive Nanopartikel. Diese weisen ein Absorptionsmaximum bei 372 nm auf und besitzen ein Emissionsmaximum bei 488 nm. Um den strahlungslosen Energietransfer zwischen den optisch aktiven Nanopartikeln und dem Fluorophor-fusionierten, amphiphilen Protein zu beweisen, wurden die generierten Emulsionen mit Hilfe der Lebenszeitmessung untersucht. In der Vergleichslösung, beinhaltend ausschließlich CdS/ZnS "core-shell" Partikel haben eine hohe Quantenausbeute und die Verweilzeit des angeregten Zustands der Nanopartikel folgt einer biexponentiellen Gesetzmäßigkeit. Die Verweilzeit des angeregten Zustandes beträgt 3,2x10⁻⁸ Sekunden. Die Relaxationszeit der angeregten Nanopartikel wird durch den Zusatz des Fluorophor-fusionierten, amphiphilen Proteins auf einen Wert von 8,2x10⁻¹¹ Sekunden reduziert. Dieses Ergebnis belegt, das die Nanopartikel als Donoren fungieren. Gleichzeitig wird die Fluoreszenzintensität des Fluorophor-fusionierten, amphiphilen Proteins in der Gegenwart der Nanopartikel um den Faktor neun erhöht. Beide Ergebnisse belegen den Energietransfer von den Nanopartikeln auf das Fluorophor-fusionierte, amphiphile Protein.

**Fig. 2****:** Lebenszeitmessung des angeregten Zustands der CdS/ZnS "core-shell"-Partikel. Zu sehen ist die Verteilung der Lebenszeit des angeregten Zustands der CdS/ZnS "core-shell"-Partikel ohne Zusatz des Fluorophor-fusionierten, amphiphilen Proteins Ccg2-tRFP (gepunktete Linie) und in Gegenwart des Fluorophor-fusionierten, amphiphilen Proteins Ccg2-tRFP (durchgehende Linie).

### Ausführungsbeispiel 4: Einfluss der Donor-Konzentration auf die Fluoreszenzintensität des Akzeptors.

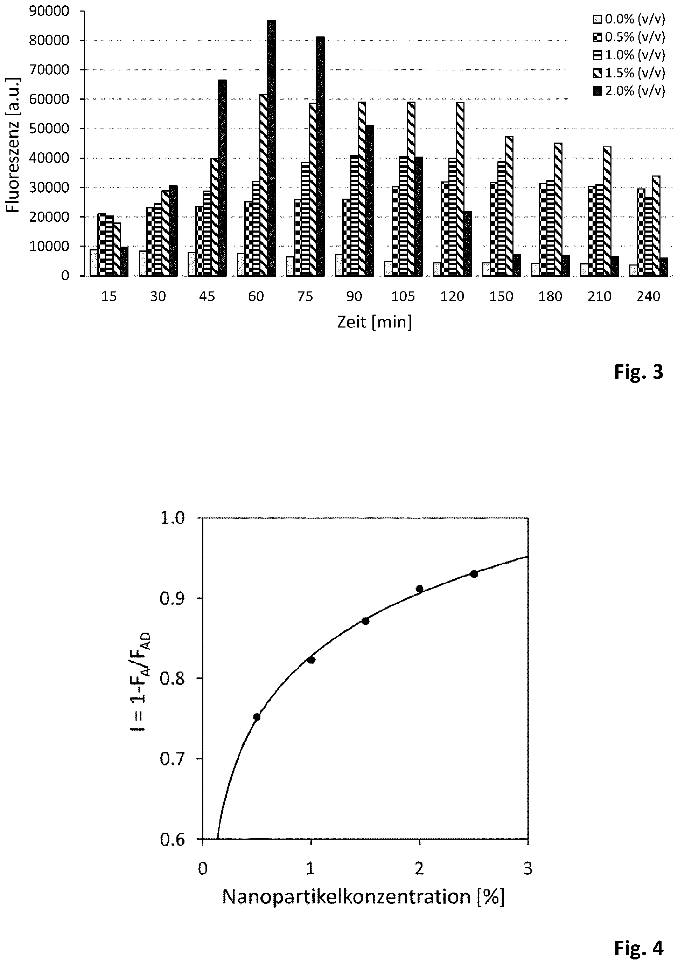

Zur Bewertung der FRET-Effektivität wurden fünf verschiedene Protein-stabilisierte Emulsionen analog zu Ausführungsbeispiel 2 erzeugt. Alle wässrigen Phasen enthielten eine finale Proteinkonzentration von 300 ng/*µ*l des Fluorophor-fusionierten, amphiphilen Proteins Ccg2-tRFP. Die organische Phase enthielt CdSe Partikel als optisch aktive Nanopartikel, die ein Absorptionsmaximum bei 368 nm aufweisen und ein Emissionsmaximum bei 488 nm aufweisen. Die erzeugten Emulsionen enthielten 0.0% bis 2.0% der CdSe Partikel. Alle Emulsionen wurden über einen Gesamtzeitraum von 4 h in einem geschlossenen System inkubiert. Alle 15 min wurden 100 *µ*l einer Probe mittels Plattenreader-basierender Fluorometrie untersucht, wobei das Fluoreszenzspektrum des Akzeptors (Ccg2-tRFP) ausgelesen wurde. Während der ersten Stunde stieg in allen Proben die Fluoreszenzintensität bis zum Erreichen eines stationären Zustandes an (s. Figur 3). Mit steigendem Donor-zu-Akzeptor-Verhältnis steigt auch die maximal erreichte Fluoreszenzintensität des Akzeptors (Ccg2-tRFP). Der resultierende Anstieg in der FRET-Effizienz (s. Figur 4) ist ein weiterer Beweis für einen wirksamen FRET.

**Fig. 3****:** Zeitabhängige Fluoreszenzintensität des Akzeptors (Ccg2-tRFP). Aufgetragen ist die Fluoreszenzintensität des Akzeptors über der Zeit für unterschiedliche Konzentrationen des Donors (CdSe-Partikel) und gleiche Konzentrationen des Akzeptors (Ccg2-tRFP).

**Fig. 4****:** Die Empfindlichkeit des FRETs. Mit steigendem Konzentration des Donors (CdSe Partikel) im Verhältnis zum Akzeptor (Ccg2-tRFP), steigt die Fluoreszenzintensität des Akzeptors (Ccg2-tRFP).

### Ausführungsbeispiel 5: Austausch des Donor-Akzeptorpaares.

Um sicherzustellen, dass man die Funktion des anregbaren Proteins und des optisch aktiven Nanopartikels auch austauschen kann, wurde eine Protein-stabilisierte Emulsionen analog zu Ausführungsbeispiel 2 mit dem Fluorophor-fusionierten, amphiphilen Protein HFBI-tRFP generiert. Die organische Phase enthielt CdSe Partikel als optisch aktive Nanopartikel, die ein Absorptionsmaximum bei 492 nm aufweisen und ein Emissionsmaximum bei 596 nm aufweisen. Die Emulsion wurde über einen Gesamtzeitraum von 4 d in einem geschlossenen System inkubiert. Alle 8 h wurden 100 µl einer Probe mittels Plattenreader-basierender Fluorometrie untersucht, wobei das Fluoreszenzspektrum des Donors (Ccg2-tRFP) und des Akzeptors (CdSe Partikel) ausgelesen wurden. Am ersten Tag konnte ausschließlich ein Emissionsspektrum des Akzeptors ohne das Auftreten einer "Schulter" im Bereich des Donors erhalten werden, welches ein Fluoreszenzmaximum bei 596 nm aufwies. Fluoreszenzmikroskopische Aufnahmen zeigten eine gleichmäßige Rotfärbung der organischen Phase ohne Auftreten einer roten Korona. Dies deutet auf eine homogene Verteilung der CdSe Partikel in der organischen Phase hin. Auf Grund des Ausbleibens einer roten Korona, kann keine Aussage über die Proteinlokalisierung getroffen werden. Am vierten Tag konnte mittels Plattenreader (Infinite M200) ausschließlich ein Spektrum für den Donor aufgenommen werden, welches ein Maximum in der Fluoreszenzintensität bei 576 nm aufweist. Fluoreszenzmikroskopische Aufnahmen zeigten eine rote Korona an der flüssig-flüssig-Phasengrenze. Dies beweist die Anlagerung des Donors (Ccg2-tRFP) an der organischen Phase der generierten Emulsion. Hingegen war nur eine schwache Rotfärbung innerhalb der organischen Phase detektierbar.

### Ausführungsbeispiel 6: Katalytischer Einsatz

Zur katalytischen Synthese von Reduktionsäquivalenten (reduziertes Nicotinamidadenindinukleotid) werden Protein-stabilisierte Emulsionen analog zu Ausführungsbeispiel 2 mit dem amphiphilen Protein ArtOLEO-LeuDH erzeugt. Das amphiphile Protein ArtOLEO-LeuDH besteht aus einem künstlich abgeleiteten Oleosin und einer C-terminal fusionierten Leucindehydrogenase. Die organische Phase enthält CdSe Partikel als optisch aktive Nanopartikel sowie einen Platin-basierenden Katalysator als Elektronmediator. Durch Einstrahlung von 150 W Xenonlicht, werden die optisch aktiven Nanopartikel in der organischen Phase angeregt. Als Folge dessen werden Elektronen über den Platin-basierenden Katalysator auf die Leucindehydrogenase in der wässrigen Phase transferiert, welche zur Reduktion von Protonen zur Verfügung stehen. Letztendlich führt die Umsetzung der Edukte L-Leucin, Wasser und Nicotinamidadenindinukleotid (oxidierte Variante) zur Generierung von reduziertem Nicotinamidadenindinukleotid, wobei Ammoniak und 4-Methyl-2-oxopentanoate als Nebenprodukte anfallen. Die reduzierte Form des Nicotinamidadenindinukleotid kann nach chromatographischer Auftrennung des Stoffgemisches über eine photometrische Messung der Absorption bei 259 nm und 339 nm nachgewiesen werden. Die reduzierte Form des Nicotinamidadenindinukleotid dient als ubiquitäres Reduktionsäquivalent für weitere enzymatische Reaktionen.

### Ausführungsbeispiel 7:

Die Interaktion zwischen dem Protein Streptavidin und dem organischen Vitamin Biotin ist eine der stärksten bekannten nichtkovalenten biologischen Bindungen. Die starke Streptavidin-Biotin-Interaktion kann durch die Anwendung in einem s.g. Zell-Surface-Display gezielt ausgenutzt werden. Durch die Behandlung von Zellen mit Biotin, wird Biotin durch Wechselwirkungen mit der Zellwand an der Zelloberfläche gebunden (Biotinylierung). In einem zweiten Schritt werden analog zum Ausführungsbeispiel 2 Protein-stabilisierte Emulsionen mit einem Fluorophor-fusionierten, amphiphilen Protein cStrep-Ccg2-eGFP erzeugt. Das Fluorophor-fusionierten, amphiphilen Protein cStrep-Ccg2-eGFP besitzt am C-Terminus die anregbare Proteindomäne eGFP und am N-Terminus die Biotin-bindende Proteindomäne cStrep. Die organische Phase enthält CdSe Partikel als optisch aktive Nanopartikel, die ein Absorptionsmaximum bei 368 nm aufweisen und ein Emissionsmaximum bei 488 nm aufweisen.

Bringt man biotinylierte Zellen und Protein-stabilisierte Emulsionen zusammen, so binden die Protein-stabilisierten Öltropfen mit dem Biotin auf der Zelloberfläche, wodurch das anregbare Protein sehr dicht an die Zellwand herangeführt wird. Durch Anregung des optisch aktiven Nanopartikels im spezifischen Wellenlängenbereich, wird der Energietransfer von dem optisch aktiven Nanopartikel auf das anregbare Protein induziert, welches im Proteinspezifischen Wellenlängenbereich emittiert. Bei der Lichtemission entsteht zugleich Wärme, welche an die Umgebung abgeführt wird. Durch die enge Anbindung des anregbaren Proteins an die Zelloberfläche der biotinylierten Zellen, kommt es zur lokalen Temperatursteigerung an der Zelloberfläche, was das Zellwachstum der biotinylierten Zellen verringert.

In der Patentbeschreibung wird folgende Nichtpatentliteratur zitiert:
Chiang, C. J.; Lin, C. C.; Lu, T. L.; Wang, H. F. Nanobiotechnology 2011, 22(41), 415102.
Clapp, A. R et al. Am. Chem. Soc. 2004, 126, 301-310.
Joensuu, J. J. et al. Plant Physiol. 2010, 152, 622-633.
Kurz, A. et al. Angew. Chem. 2006, 45, 4440-4444.
LaMer, V.K. and Dinegar, R.H. J. Am. Chem. Soc. 1950, 72, 4847-4854.
Mattoussi, H. et al. J. Lab. Autom. 2004, 9, 28-32.
Nicolini, C. et al. J. Am. Chem. Soc. 2005, 128, 192-201.
Oritz, A. et al. Biopolymers 2000, 53(6), 455-466.
Soekarjo, M. et al. Biochemistry US 1996, 35(4), 1232-1241.
Vance, V. B. and Huang, A. H. C., J. Biol. Chem., 1987, 262(23), 11275-11279.
Zampieri, F.; Wösten, H. A. B.; Scholtmeijer, K. Materials 2010, 3(9), 4607-4625.

## Patentansprüche

1. System zum strahlungslosen Energietransfer über eine Phasengrenze eines flüssig-flüssig-Mehrphasensystems mit einer organischen Phase und einer wässrigen Phase enthaltend:
a) ein erstes Molekül, welches für einen Förster-Resonanz-Energie-Transfer (FRET) geeignet ist und sich in der organischen Phase befindet, und
b) ein amphiphiles Protein, welches eine hydrophile Domäne, die in die wässrige Phase zeigt, und eine hydrophobe Domäne, die in die organische Phase zeigt, aufweist und welches mit einem zweiten Molekül markiert ist,
wobei das zweite Molekül ein anregbares Protein ist,
welches für einen Förster-Resonanz-Energie-Transfer mit dem ersten Molekül geeignet ist und in die wässrige Phase zeigt,
wobei das zweite Molekül an die hydrophile Domäne gebunden ist oder diese bildet,
wobei die organische Phase ein organisches, nicht mit Wasser mischbares Lösungsmittel oder Öl enthält,
wobei das erste Molekül und das zweite Molekül so ausgewählt sind, dass beide Moleküle ein Donor/Akzeptorpaar bilden, welches einen Förster-Resonanzenergietransfer (FRET) ermöglicht, wobei der Donor und der Akzeptor des Donor/Akzeptorpaares durch eine flüssig-flüssig-Phasengrenze voneinander separiert sind.

2. System nach Anspruch 1, **dadurch gekennzeichnet, dass** die organische Phase optisch aktive Nanopartikel als erstes Molekül enthält.

3. System nach Anspruch 1 oder 2, **dadurch gekennzeichnet, dass** das zweite Molekül als Fusionsprotein mit dem amphiphilen Protein vorliegt.

4. System nach einem der Ansprüche 1 bis 3, **dadurch gekennzeichnet, dass** das erste Molekül den FRET-Donor und das zweite Molekül den FRET-Akzeptor bildet.

5. System nach einem der Ansprüche 1 bis 4, **dadurch gekennzeichnet, dass** das optisch aktive Nanopartikel als erstes Molekül den FRET-Donor und das anregbare Protein als zweites Molekül den FRET-Akzeptor bildet.

6. Verwendung eines Systems nach einem der Ansprüche 1 bis 5 oder amphiphilen Proteins wie in einem der Ansprüche 1 bis 5 definiert zur Signalübertragung und Signalumwandlung an Phasengrenzen in einem flüssig-flüssig-Mehrphasensystem, insbesondere zur Detektion der Integrität der Phasengrenzen.

7. System nach einem der Ansprüche 1 bis 5, **dadurch gekennzeichnet, dass** das amphiphile Protein an eine Domäne mit enzymatischer Aktivität gekoppelt ist, bevorzugt dadurch, dass die Domäne als weiterer Fusionspartner vorliegt.

8. Verwendung eines Systems nach einem der Ansprüche 1 bis 5 oder 7 oder eines hydrophoben amphiphilen Proteins wie in einem der Ansprüche 1 bis 5 oder 7 definiert zur Katalyse von Reaktionen an Phasengrenzen in einem flüssig-flüssig-Mehrphasensystem.

9. System nach einem der Ansprüche 1 bis 5 oder 7, **dadurch gekennzeichnet, dass** das amphiphile Protein an ein Molekül mit spezifischen Bindungseigenschaften, bevorzugt ausgewählt aus Antikörpern, Aptameren und Liganden, gekoppelt ist, die spezifisch an Oberflächenmoleküle von Zellen oder Mikroorganismen binden.

## Claims

1. A system for radiation-free energy transfer across a phase boundary of a liquid-liquid multiphase system having an organic phase and an aqueous phase, comprising:
a) a first molecule, which is suitable for a Förster resonance energy transfer (FRET) and is in the organic phase, and
b) an amphiphilic protein, which has a hydrophilic domain that protrudes into the aqueous phase and a hydrophobic domain that protrudes into the organic phase and which is marked with a second molecule, wherein the second molecule is an excitable protein, which is suitable for a Förster resonance energy transfer with said first molecule and protrudes into the aqueous phase wherein the second module is bound to the hydrophilic domain or forms said domain,
wherein the organic phase contains an organic solvent or oil which is not miscible with water, wherein the first molecule and the second molecule are selected such that both molecules form a donor-acceptor pair that enables a Förster resonance energy transfer (FRET), wherein the donor and the acceptor of the donor-acceptor pair are separated by a liquid-liquid phase boundary.

2. System according to claim 1, **characterised in that** the organic phase contains optically active nanoparticles as the first molecule.

3. System according to claim 1 or 2, **characterised in that** the second molecule is present as a fusion protein with the amphiphilic protein.

4. System according to any one of claims 1 to 3, **characterised in that** the first molecule forms the FRET donor and the second molecule forms the FRET acceptor.

5. System according to any one of claims 1 to 4, **characterised in that** the optically active nanoparticles form the FRET donor as the first molecule and the excitable protein forms the FRET acceptor as the second molecule.

6. Use of a system according to any one of claims 1 to 5 or of amphiphilic protein as defined in any one of claims 1 to 5 for signal transmission and signal conversion at phase boundaries in a liquid-liquid multiphase system, in particular for detecting the integrity of the phase boundaries.

7. System according to and one of claims 1 to 5, **characterised in that** the amphiphilic protein is coupled to a domain with enzymatic activity, preferably because the domain is present as a further fusion partner.

8. Use of a system according to any one of claims 1 to 5 or 7 or of a hydrophobic amphiphilic protein as defined in any one of claims 1 to 5 or 7 for the catalysis of reactions at phase boundaries in a liquid-liquid multiphase system.

9. System according to any one of claims 1 to 5 or 7, **characterised in that** the amphiphilic protein is coupled to a molecule having specific binding properties, preferably selected from antibodies, aptamers and ligands, which bind selectively to surface molecules of cells or microorganisms.

## Revendications

1. Système de transfert d'énergie sans rayonnement via une limite de phase d'un système multiphasé liquide-liquide avec une face organique et une phase aqueuse contenant :
a) une première molécule qui est adaptée pour un transfert d'énergie à résonance Förster (FRET) et se trouve en phase organique et
b) une protéine amphiphile qui présente un domaine hydrophile qui est orienté vers la phase aqueuse et un domaine hydrophobe qui est orienté vers la face organique, et qui est marqué par une seconde molécule,
la seconde molécule étant une protéine excitable
qui est adaptée pour un transfert d'énergie à résonance Förster et qui est orientée vers la phase aqueuse,
la seconde molécule étant liée au domaine hydrophile ou constituant celui-ci,
la phase organique contenant un solvant organique non miscible à l'eau ou de l'huile,
la première molécule et la seconde molécule étant sélectionnées de manière à ce que les deux molécules forment une paire donneur/receveur qui permet un transfert d'énergie à résonance Förster (FRET), le donneur et le receveur de la paire donneur/receveur étant séparés l'un de l'autre par une limite de phase liquide-liquide.

2. Système selon la revendication 1, **caractérisé en ce que** la phase organique contient des nanoparticules optiquement actives comme première molécule.

3. Système selon la revendication 1 ou 2, **caractérisé en ce que** la seconde molécule est présente sous forme de protéine de fusion avec la protéine amphiphile.

4. Système selon une des revendications 1 à 3, **caractérisé en ce que** la première molécule constitue le donneur FRET et la seconde molécule le receveur FRET.

5. Système selon une des revendications 1 à 4, **caractérisé en ce que** la nanoparticule optiquement active en tant que première molécule constitue le donneur FRET et la protéine excitable en tant que seconde molécule le receveur FRET.

6. Utilisation d'un système selon une des revendications 1 à 5 ou d'une protéine amphiphile comme définie dans une des revendications 1 à 5 pour la transmission de signaux et la conversion de signaux à des limites de phase dans un système multiphasé liquide-liquide, en particulier pour la détection de l'intégrité des limites de phase.

7. Système selon une des revendications 1 à 5, **caractérisé en ce que** la protéine amphiphile est couplée à un domaine à activité enzymatique, de préférence du fait que le domaine est présent sous forme d'autre partenaire de fusion.

8. Utilisation d'un système selon une des revendications 1 à 5 ou 7 ou d'une protéine amphiphile hydrophobe comme définie dans une des revendications 1 à 5 ou 7 pour la catalyse de réactions à des limites de phase dans un système multiphasé liquide-liquide.

9. Système selon une des revendications 1 à 5 ou 7, **caractérisé en ce que** la protéine amphiphile est couplée à une molécule à propriétés de liaison spécifiques, sélectionnée de préférence parmi les anticorps, les aptamères et les ligands qui se lient spécifiquement à des molécules superficielles de cellules ou de micro-organismes.
